# EUROPEAN PATENT APPLICATION

(11) **EP 4 279 509 A1**
(43) Date of publication of application: **22.11.2023**
(21) Application number: 22739160.4
(22) Date of filing: 17.01.2022
(51) Int. Cl.: C07K 16/28, A61K 39/00, A61K 39/395

(54) **GARP PROTEIN ANTIBODY AND APPLICATION THEREOF**

(30) Priority: 18.01.2021 CN 202110065290
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: ZHANG, Jianjian, Shanghai 201203 (CN); PAN, Zhongzong, Shanghai 201203 (CN); YANG, Xinxiu, Shanghai 201203 (CN); LIU, Xiaowu, Shanghai 201203 (CN); YANG, Lu, Shanghai 201203 (CN); LIU, Peipei, Shanghai 201203 (CN); CAO, Xiaodan, Shanghai 201203 (CN); DENG, Sujun, Shanghai 201203 (CN); WANG, Xueping, Shanghai 201203 (CN)
(74) Representative: Bianchetti & Minoja with Trevisan & Cuonzo IPS SRL
(86) International application number: PCT/CN2022/072265
(87) International publication number: WO 2022/152285

(57) **Abstract**

Provided is a GARP protein antibody, and a cell comprising or expressing the GARP protein antibody. The GARP protein antibody binds to the human GARP/human TGF-β1 complex with a K_{D} value of about 1.0E-12 or less. Also provided are a pharmaceutical combination comprising the GARP protein antibody and an immune checkpoint inhibitor and use thereof.

## Description

### FIELD OF THE INVENTION

The present application relates to the field of biomedicine, and in particular to a GARP protein antibody and use thereof.

### BACKGROUND OF THE INVENTION

Regulatory T cells (Tregs) are closely associated with autoimmune diseases and tumorigenesis, the abnormal activity of which can cause autoimmune diseases or tumorigenesis. Treg cells as a current research hotspot in the field of immunology have two characteristics of low immune response and immunosuppression, and play a role in negative immunoregulation by inhibiting an immune system in an "active" mode. Depending on the source and mechanism of action, Tregs can be classified into naturally occurring CD4+CD25+Tregs (natural regulatory T cells, nTregs) and induced adaptive regulatory T cells (aTregs or iTregs).

As an 80 kDa type I membrane glycoprotein, GARP (human glycoprotein A repetitions predominant) protein is expressed on megakaryocytes, platelets and activated regulatory T cells (Treg cells), belonging to the leucine-rich repeat-containing protein family. TGF-β forms a cage structure with the inactive binding protein LAP in an inactive state, which can be activated by GARP, integrins, proteases or other substrates to form active TGF-β, which activates cellular signaling by binding to receptors via autocrine or paracrine. TGF-β signaling is thought to be primarily involved in the development of tumors resistance to checkpoint blockade therapies. For example, immunosuppressive cells and immunosuppressive factors are increased by TGF-β in the tumor microenvironment.

As an immunosuppressive cytokine, TGF-β can inhibit the immune response broadly through different mechanisms. TGF-β reduces the differentiation and function of Th1, Th2 cells, and cytotoxic T lymphocytes (CTLs), all of which provide important anti-tumor effects. TGF-β also enhances immune tolerance and tumor evasion by regulating the number and function of regulatory T (Treg) cells. In addition, TGF-β regulates immune cell fate by inhibiting or stimulating cell proliferation, thereby affecting the development of the thymus and peripheral T cells.

Currently, all TGF-β isoforms are neutralized by most clinical antibodies, which has large safety issues. Therapies targeting the GARP-TGF-β1 complex can inhibit the release of active TGF-β1 from Treg, thereby increasing the anti-tumor activity of PD-1/PD-L1. At present, there is an urgent need for a novel antibody against the GARP-TGF-β1 complex, which has fewer side effects, high safety, and less adverse effects on the tumor microenvironment than broadly block all TGF-β isoforms.

### SUMMARY OF THE INVENTION

The present application provides an isolated antigen-binding protein having one or more of the following properties: 1) binding to the human GARP/human TGF-β1 complex in an Octet assay with a KD value of about 1.0E-12 or less; 2) not binding to cells expressing only human GARP or cells expressing only human TGF-β1; 3) inhibiting the SMAD2 phosphorylation in Treg cells; 4) inhibiting the release of TGF-β1 from HEK293T cells expressing human GARP/human TGF-β1; 5) preventing Treg cells from inhibiting the release of interleukin-2 (IL-2) and interferon- γ (IFN- γ) from human peripheral blood mononuclear cells (PBMC); and 6) blocking the inhibitory effect of Treg cells on GvHD induced by the human PBMCs in a human PBMC transplanted mice GvHD model. In one aspect, the present application provides an isolated antigen-binding protein comprising HCDR3 comprising an amino acid sequence as set forth in SEQ ID NO: 4.

In some embodiments, the isolated antigen-binding protein comprises HCDR2 comprising an amino acid sequence as set forth in SEQ ID NO: 3.

In some embodiments, the isolated antigen-binding protein comprises HCDR1 comprising an amino acid sequence as set forth in SEQ ID NO: 2.

In some embodiments, the isolated antigen-binding protein comprises a heavy chain variable region VH comprising the HCDR1 comprising an amino acid sequence as set forth in SEQ ID NO: 2, the HCDR2 comprising an amino acid sequence as set forth in SEQ ID NO: 3, and the HCDR3 comprising an amino acid sequence as set forth in SEQ ID NO: 4.

In some embodiments, the isolated antigen-binding protein comprises H-FR1, wherein the C-terminus of the H-FR1 is directly or indirectly linked to the N-terminus of the HCDR1, and the H-FR1 comprises an amino acid sequence as set forth in SEQ ID NO: 46.

In some embodiments, the H-FR1 of the antigen-binding protein comprises an amino acid sequence as set forth in any one of SEQ ID NO: 5 and SEQ ID NO: 22.

In some embodiments, the isolated antigen-binding protein comprises H-FR2, wherein the H-FR2 is located between the HCDR1 and the HCDR2, and the H-FR2 comprises an amino acid sequence as set forth in SEQ ID NO: 47.

In some embodiments, the H-FR2 of the antigen-binding protein comprises an amino acid sequence as set forth in any one of SEQ ID NO: 6 and SEQ ID NO: 23.

In some embodiments, the isolated antigen-binding protein comprises H-FR3, wherein the H-FR3 is located between the HCDR2 and the HCDR3, and the H-FR3 comprises an amino acid sequence as set forth in SEQ ID NO: 48.

In some embodiments, the H-FR3 of the antigen-binding protein comprises an amino acid sequence as set forth in any one of SEQ ID NO: 7 and SEQ ID NO: 24.

In some embodiments, the isolated antigen-binding protein comprises H-FR4, wherein the N-terminus of the H-FR4 is directly or indirectly linked to the C-terminus of the HCDR3, and the H-FR4 comprises an amino acid sequence as set forth in SEQ ID NO: 49.

In some embodiments, the H-FR4 of the antigen-binding protein comprises an amino acid sequence as set forth in any one of SEQ ID NO: 8 and SEQ ID NO: 25.

In some embodiments, the isolated antigen-binding protein comprises H-FR1 comprising an amino acid sequence as set forth in SEQ ID NO: 46, H-FR2 comprising an amino acid sequence as set forth in SEQ ID NO: 47, H-FR3 comprising an amino acid sequence as set forth in SEQ ID NO: 48, and H-FR4 comprising an amino acid sequence as set forth in SEQ ID NO: 49.

In some embodiments, the isolated antigen-binding protein comprises H-FR1 comprising an amino acid sequence as set forth in any one of SEQ ID NO: 5 and SEQ ID NO: 22, H-FR2 comprising an amino acid sequence as set forth in any one of SEQ ID NO: 6 and SEQ ID NO: 23, H-FR3 comprising an amino acid sequence as set forth in any one of SEQ ID NO: 7 and SEQ ID NO: 24, and H-FR4 comprising an amino acid sequence as set forth in any one of SEQ ID NO: 8 and SEQ ID NO: 25.

In some embodiments, the H-FR1, H-FR2, H-FR3, and H-FR4 of the antigen-binding protein comprise amino acid sequences selected from any one of the groups consisting of:
a) H-FR1: SEQ ID NO: 5, H-FR2: SEQ ID NO: 6, H-FR3: SEQ ID NO: 7, and H-FR4: SEQ ID NO: 8;
b) H-FR1: SEQ ID NO: 22, H-FR2: SEQ ID NO: 23, H-FR3: SEQ ID NO: 24, and H-FR4: SEQ ID NO: 25.

In some embodiments, the isolated antigen-binding protein comprises a heavy chain variable region VH comprising an amino acid sequence as set forth in SEQ ID NO: 50.

In some embodiments, the VH of the antigen-binding protein comprises an amino acid sequence as set forth in any one of SEQ ID NO: 1 and SEQ ID NO: 21.

In some embodiments, the isolated antigen-binding protein comprises LCDR3 comprising an amino acid sequence as set forth in SEQ ID NO: 12.

In some embodiments, the isolated antigen-binding protein comprises LCDR2 comprising an amino acid sequence as set forth in SEQ ID NO: 45.

In some embodiments, the isolated antigen-binding protein comprises LCDR2 comprising an amino acid sequence as set forth in SEQ ID NO: 11 and SEQ ID NO: 28.

In some embodiments, the isolated antigen-binding protein comprises LCDR1 comprising an amino acid sequence as set forth in SEQ ID NO: 44.

In some embodiments, the isolated antigen-binding protein comprises LCDR1 comprising an amino acid sequence as set forth in SEQ ID NO: 10 and SEQ ID NO: 27.

In some embodiments, the isolated antigen-binding protein comprises a light chain variable region VL which comprises the LCDR1 comprising an amino acid sequence as set forth in SEQ ID NO: 44, the LCDR2 having amino acid sequence as set forth in SEQ ID NO: 45, and the LCDR3 comprising an amino acid sequence as set forth in SEQ ID NO: 12.

In some embodiments, the isolated antigen-binding protein comprises a light chain variable region VL comprising the LCDR1 comprising an amino acid sequence as set forth in any one of SEQ ID NO: 10 and SEQ ID NO: 27, the LCDR2 comprising an amino acid sequence as set forth in any one of SEQ ID NO: 11 and SEQ ID NO: 28, and the LCDR3 comprising an amino acid sequence as set forth in SEQ ID NO: 12.

In some embodiments, the LCDR1, LCDR2, and LCDR3 in the isolated antigen-binding protein have an amino acid sequence selected from any one of the groups consisting of:
a) LCDR1: SEQ ID NO: 10, LCDR2: SEQ ID NO: 11, and LCDR3: SEQ ID NO: 12;
b) LCDR1: SEQ ID NO: 27, LCDR2: SEQ ID NO: 28, and LCDR3: SEQ ID NO: 12.

In some embodiments, the isolated antigen-binding protein comprises L-FR1, wherein the C-terminus of the L-FR1 is directly or indirectly linked to the N-terminus of the LCDR1, and the L-FR1 comprises an amino acid sequence as set forth in SEQ ID NO: 52.

In some embodiments, the L-FR1 of the antigen-binding protein comprises an amino acid sequence as set forth in any one of SEQ ID NO: 13 and SEQ ID NO: 29.

In some embodiments, the isolated antigen-binding protein comprises L-FR2, wherein the L-FR2 is located between the LCDR1 and the LCDR2, and the L-FR2 comprises an amino acid sequence as set forth in SEQ ID NO: 14.

In some embodiments, the isolated antigen-binding protein comprises L-FR3, wherein the L-FR3 is located between the LCDR2 and the LCDR3, and the L-FR3 comprises an amino acid sequence as set forth in SEQ ID NO: 53.

In some embodiments, the L-FR3 of the antigen-binding protein comprises an amino acid sequence as set forth in any one of SEQ ID NO: 15 and SEQ ID NO: 30.

In some embodiments, the isolated antigen-binding protein comprises L-FR4, wherein the N-terminus of the L-FR4 is directly or indirectly linked to the C-terminus of the LCDR3, and the L-FR4 comprises an amino acid sequence as set forth in SEQ ID NO: 54.

In some embodiments, the L-FR4 of the antigen-binding protein comprises an amino acid sequence as set forth in any one of SEQ ID NO: 16 and SEQ ID NO: 31.

In some embodiments, the isolated antigen-binding protein comprises L-FR1 comprising an amino acid sequence as set forth in SEQ ID NO: 52, L-FR2 comprising an amino acid sequence as set forth in SEQ ID NO: 14, L-FR3 comprising an amino acid sequence as set forth in SEQ ID NO: 53, and L-FR4 comprising an amino acid sequence as set forth in SEQ ID NO: 54.

In some embodiments, the isolated antigen-binding protein comprises L-FR1 comprising an amino acid as sequence as set forth in any one of SEQ ID NO: 13 and SEQ ID NO: 29, L-FR2 comprising an amino acid sequence as set forth in SEQ ID NO: 14, L-FR3 comprising an amino acid sequence as set forth in any one of SEQ ID NO: 15 and SEQ ID NO: 30, and L-FR4 comprising an amino acid sequence as set forth in any one of SEQ ID NO: 16 and SEQ ID NO: 31.

In some embodiments, the L-FR1, L-FR2, H-FR3, and L-FR4 of the antigen-binding protein comprise amino acid sequences selected from any one of the groups consisting of:
a) L-FR1: SEQ ID NO: 13, L-FR2: SEQ ID NO: 14, L-FR3: SEQ ID NO: 15, and H-FR4: SEQ ID NO: 16;
b) L-FR1: SEQ ID NO: 29, L-FR2: SEQ ID NO: 14, L-FR3: SEQ ID NO: 30, and H-FR4: SEQ ID NO: 31.

In some embodiments, the isolated antigen-binding protein comprises a light chain variable region VL comprising an amino acid sequence as set forth in SEQ ID NO: 51.

In some embodiments, the VL of the antigen-binding protein comprises an amino acid sequence as set forth in any one of SEQ ID NO: 9 and SEQ ID NO: 26.

In some embodiments, the isolated antigen-binding protein comprises a heavy chain constant region comprising an IgG-derived constant region or an IgY-derived constant region.

In some embodiments, the antigen-binding protein heavy chain constant region comprises a human IgG4-derived constant region.

In some embodiments, the antigen-binding protein heavy chain constant region comprises an amino acid sequence as set forth in SEQ ID NO: 17.

In some embodiments, the isolated antigen-binding protein comprises a light chain constant region, and wherein the antibody light chain constant region comprises an Igκ-derived constant region or an Igλ-derived constant region.

In some embodiments, the antigen-binding protein light chain constant region comprises a human Igκ-derived constant region.

In some embodiments, the antigen-binding protein light chain constant region comprises an amino acid sequence as set forth in SEQ ID NO: 18.

In some embodiments, the isolated antigen-binding protein comprises a heavy chain HC comprising an amino acid sequence as set forth in any one of SEQ ID NO: 19 and SEQ ID NO: 32.

In some embodiments, the isolated antigen-binding protein comprises a light chain LC comprising an amino acid sequence as set forth in any one of SEQ ID NO: 20 and SEQ ID NO: 33.

In some embodiments, the isolated antigen-binding protein comprises an HC comprising an amino acid sequence as set forth in any one of SEQ ID NO: 19 and SEQ ID NO: 32 and an LC comprising an amino acid sequence as set forth in any one of SEQ ID NO: 20 and SEQ ID NO: 33.

In some embodiments, the HC and LC of the antigen-binding protein comprise amino acid sequences selected from any one of the groups consisting of:
a) HC: SEQ ID NO: 19 and LC: SEQ ID NO: 20;
b) HC: SEQ ID NO: 32 and LC: SEQ ID NO: 33.

In some embodiments, the isolated antigen-binding protein comprises an antibody or an antigen-binding fragment thereof.

In some embodiments, the antigen-binding fragment comprises Fab, Fab', F(ab)2, Fv fragments, F(ab')2, scFv, di-scFv, VHH and/or dAb.

In some embodiments, the antibody is selected from the group consisting of: monoclonal antibodies, single chain antibodies, murine antibodies, chimeric antibodies, humanized antibodies, and fully human antibodies.

In another aspect, the present application provides a polypeptide comprising the isolated antigen-binding protein.

In another aspect, the present application provides an immunoconjugate comprising the isolated antigen-binding protein or the polypeptide.

In some embodiments, the immunoconjugate also includes a pharmaceutically acceptable therapeutic agent.

In some embodiments, the therapeutic agent is selected from the group of a cytotoxic agent and a cytostatic agent.

In another aspect, the present application provides an isolated nucleic acid molecule encoding the isolated antigen-binding protein, or the polypeptide.

In another aspect, the present application provides a vector comprising the isolated nucleic acid molecule.

In another aspect, the present application provides a cell comprising the isolated antigen-binding protein, the polypeptide, the immunoconjugate, the isolated nucleic acid molecule, and/or the vector.

In another aspect, the present application provides a method of preparing the isolated antigen-binding protein or the polypeptide, the method comprising culturing the cell under conditions capable of expressing the isolated antigen-binding protein or the polypeptide.

In another aspect, the present application provides a pharmaceutical composition comprising the isolated antigen-binding protein, the polypeptide, the immunoconjugate, the isolated nucleic acid molecule, the vector, the cell, and/or a pharmaceutically acceptable adjuvant and/or excipient.

In another aspect, the present application provides a pharmaceutical combination comprising the isolated antigen-binding protein and an immune checkpoint inhibitor.

In some embodiments, the immune checkpoint inhibitor comprises a substance that inhibits the interaction of PD-1/PD-L1.

In some embodiments, the immune checkpoint inhibitor is selected from the group consisting of: PD-1/PD-L1 blocking agents, PD-1 antagonists, PD-L1 antagonists, PD-1 inhibitors, and PD-L1 inhibitors.

In some embodiments, the immune checkpoint inhibitor comprises an anti-PD-L1 antibody.

In some embodiments, the anti-PD-L1 antibody comprises HCDR3 comprising an amino acid sequence as set forth in SEQ ID NO: 37.

In some embodiments, the anti-PD-L1 antibody comprises HCDR2 comprising an amino acid sequence as set forth in SEQ ID NO: 36.

In some embodiments, the anti-PD-L1 antibody comprises HCDR1 comprising an amino acid sequence as set forth in SEQ ID NO: 35.

In some embodiments, the anti-PD-L1 antibody comprises a heavy chain variable region VH comprising HCDR1 comprising an amino acid sequence as set forth in SEQ ID NO: 35, HCDR2 comprising an amino acid sequence as set forth in SEQ ID NO: 36, and HCDR3 comprising an amino acid sequence as set forth in SEQ ID NO: 37.

In some embodiments, the anti-PD-L1 antibody comprises a heavy chain variable region VH comprising an amino acid sequence as set forth in SEQ ID NO: 34.

In some embodiments, the anti-PD-L1 antibody comprises LCDR3 comprising an amino acid sequence as set forth in SEQ ID NO: 41.

In some embodiments, the anti-PD-L1 antibody comprises LCDR2 comprising an amino acid sequence as set forth in SEQ ID NO: 40.

In some embodiments, the anti-PD-L1 antibody comprises LCDR1 comprising an amino acid sequence as set forth in SEQ ID NO: 39.

In some embodiments, the anti-PD-L1 antibody comprises a light chain variable region VL comprising LCDR1 comprising an amino acid sequence as set forth in SEQ ID NO: 39, LCDR2 comprising an amino acid sequence as set forth in SEQ ID NO: 40, and LCDR3 comprising an amino acid sequence as set forth in SEQ ID NO: 41.

In some embodiments, the anti-PD-L1 antibody comprises a light chain variable region VL comprising an amino acid sequence as set forth in SEQ ID NO: 38.

In some embodiments, the anti-PD-L1 antibody includes Atezolizumab.

In some embodiments, the pharmaceutical combination can be a pharmaceutical composition.

In another aspect, the present application provides a kit comprising the pharmaceutical combination.

In another aspect, the present application provides the isolated antigen-binding protein, the polypeptide, the immunoconjugate, the isolated nucleic acid molecule, the vector, the cell, and/or the pharmaceutical composition for the prevention, remission, and/or treatment of a tumor.

In some embodiments, the tumor includes a solid tumor.

In some embodiments, the tumor includes a tumor associated with the expression of GARP.

In some embodiments, the tumor includes a melanoma, a breast tumor, and/or a lung tumor.

In another aspect, the present application provides the pharmaceutical combination and/or the kit for the prevention, remission, and/or treatment of a tumor.

In some embodiments, the tumor includes a solid tumor.

In some embodiments, the tumor includes a tumor associated with the expression of GARP.

In some embodiments, the tumor includes a melanoma, a breast tumor, and/or a lung tumor.

In another aspect, the present application provides a use of the isolated antigen-binding protein, the polypeptide, the immunoconjugate, the isolated nucleic acid molecule, the vector, the cell, and/or the pharmaceutical composition in the manufacture of a medicament for the prevention, remission, and/or treatment of a tumor.

In some embodiments, the tumor includes a solid tumor, a breast tumor, and/or a lung tumor. In some embodiments, the tumor includes a tumor associated with the expression of GARP.

In some embodiments, the tumor includes a melanoma.

In another aspect, the present application provides a use of the pharmaceutical combination and/or the kit in the manufacture of a medicament for the prevention, remission, and/or treatment of a tumor.

In some embodiments, the tumor includes a solid tumor.

In some embodiments, the tumor includes a tumor associated with the expression of GARP.

In some embodiments, the tumor includes a melanoma, a breast tumor, and/or a lung tumor.

In another aspect, the present application provides a method for the prevention and/or treatment of a disease or disorder comprising administering to a subject in need thereof an effective amount of the isolated antigen-binding protein, the isolated nucleic acid molecule, the vector, the cell, and the pharmaceutical composition, wherein the disease or disorder includes a tumor.

In some embodiments, the tumor includes a solid tumor.

In some embodiments, the tumor includes a tumor associated with the expression of GARP.

In some embodiments, the tumor includes a melanoma, a breast tumor, and/or a lung tumor.

In another aspect, the present application provides a method for the prevention and/or treatment of a disease or disorder comprising administering to a subject in need thereof an effective amount of the pharmaceutical combination, wherein the disease or disorder includes a tumor.

In some embodiments, the tumor includes a solid tumor.

In some embodiments, the tumor includes a tumor associated with the expression of GARP.

In some embodiments, the tumor includes a melanoma, a breast tumor, and/or a lung tumor.

Other aspects and advantages of the present application will become readily apparent to those skilled in the art from the following detailed description. Only exemplary embodiments of the present application are shown and described in the following detailed description. As will be recognized by those skilled in the art, the contents of the present application enable those skilled in the art to make modifications to the specific embodiments disclosed without departing from the spirit and scope of the present application to which the present application pertains. Accordingly, the drawings and description herein are to be regarded as illustrative in nature, and not as restrictive.

### BRIEF DESCRIPTION OF THE DRAWINGS

Specific features of the present application to which the present application relates are as set forth in the appended claims. The features and advantages of the present application to which the present application relates will be better understood by reference to the exemplary embodiments and drawings described in detail below. A brief description of the drawings is as follows:
Figs. 1A-1D show the antigen-binding activity of the antigen-binding proteins described herein;
Fig. 2 shows the results of blocking experiments with an exemplary murine antibody 8H2D7B3 of the present application;
Figs. 3A-3B show the effect of an exemplary murine antibody 8H2D7B3 of the present application on the SMAD2 phosphorylation in Treg cells;
Fig. 4 shows the effect of an exemplary JYB1907hz18 antibody of the present application on the SMAD2 phosphorylation in Treg cells;
Fig. 5 shows the effect of an exemplary JYB1907hz18 antibody of the present application on the release of TGF-β1 from HEK293T cells;
Figs. 6A-6B show the effect of an exemplary JYB1907hz18 antibody of the present application on the release of IL-2 and IFN-γ from PBMC;
Fig. 7A shows a pharmacokinetic (PK) profile of an exemplary JYB1907hz18 antibody of the present application in a humanized FcRn mouse model;
Fig. 7B shows a pharmacokinetic (PK) profile of the MHG8 antibody described herein in a humanized FcRn mouse model;
Figs. 8A-8B show the results of a study of an exemplary JYB1907hz18 antibody of the present application in a mouse GvHD model;
Fig. 9 shows the results of a pharmacodynamic testing of an exemplary JYB1907hz18 antibody of the present application in a mouse melanoma model;
Fig. 10 shows the results of a pharmacodynamic testing of an exemplary JYB1907hz18 antibody of the present application in a mouse breast cancer model; and
Fig. 11 shows the results of a pharmacodynamic testing of an exemplary JYB1907hz18 antibody of the present application in a mouse lung squamous cell carcinoma model.

### DETAILED DESCRIPTION OF THE INVENTION

The embodiments of the invention of the present application is illustrated by particular examples. Other advantages and effects of the present application will become readily apparent to those skilled in the art from disclosure of the present specification.

### Definition of Terms

In the present application, the term "isolated" generally refers to a product obtained from a natural state by artificial means. If an "isolated" substance or component occurs in nature, it may be altered from its natural environment, or the substance may be isolated from its natural environment, or both. For example, an unisolated polynucleotide or polypeptide naturally occurs in a living animal and the same polynucleotide or polypeptide isolated from its natural state in high purity is the to be isolated. The term "isolated" does not exclude the admixture of artificial or synthetic substances, nor the presence of other impure substances which do not affect the activity of the substance.

In the present application, the term "antigen-binding protein" generally refers to a polypeptide molecule capable of specifically recognizing and/or neutralizing a particular antigen. In the present application, the term "antigen-binding protein" may include an "antibody" or an "antigen-binding fragment". For example, the antibody may comprise an immunoglobulin composed of at least two heavy (H) chains and two light (L) chains interconnected by disulfide bonds, and may include any molecule comprising an antigen-binding portion thereof. The term "antibody" may include monoclonal antibodies, antibody fragments, or antibody derivatives, including, but not limited to, murine antibodies, human antibodies (fully human antibodies), humanized antibodies, chimeric antibodies, single chain antibodies (e.g., scFv), and antibody fragments that bind to an antigen (e.g., Fab, Fab', and (Fab)2 fragments). The term "antibody" may also include all recombinant forms of antibodies, such as antibodies expressed in prokaryotic cells, non-glycosylated antibodies, and any antigen-binding antibody fragments and derivatives thereof described herein. Each heavy chain may be composed of a heavy chain variable region and a heavy chain constant region. Each light chain may be composed of a light chain variable region and a light chain constant region. The VH and VL regions can be further distinguished as hypervariable regions called complementarity determining regions (CDRs), interspersed with more conserved regions called framework regions (FRs). Each VH and VL may be composed of three CDRs and four FRs, which may be arranged from amino-terminus to carboxy-terminus in the following order: FR1, CDR1, FR2, CDR2, FR3, CDR3, and FR4. The variable regions of the heavy and light chains contain binding domains that interact with an antigen (e.g., human GARP). The constant region of an antibody can mediate the binding of the immunoglobulin to host tissues or factors, including various cells of the immune system (e.g., effector cells) and the first component of the classical complement system (Clq). The exact boundaries of the CDRs have been defined differently for different systems. The system described by Kabat (Kabat et al., Sequences of Proteins of Immunological Interest (National Institutes of Health, Bethesda, Md. (1987) and (1991)) provides not only an unambiguous residue numbering system applicable to any variable region of an antigen-binding fragment, but also provides precise residue boundaries defining CDRs. These CDRs may be referred to as Kabat CDRs. Chothia and co-workers (Chothia and Lesk, J.Mol.Biol.196: 901-917(1987) and Chothia et al., Nature 342: 877-883(1989)) found that certain sub-portions within Kabat CDRs adopt nearly identical conformations in the backbone of a peptide despite the large diversity at the amino acid sequence level. These sub-portions are designated as L1, L2, and L3 or H1, H2, and H3, where "L" and "H" refer to the light chain and heavy chain regions, respectively. These regions may be referred to as Chothia CDRs, which have boundaries that overlap with Kabat CDRs. Other boundaries that define CDRs and overlap with Kabat CDRs have been described by Padlan (FASEB J.9: 133-139 (1995)) and MacCallum (J Mol Biol 262(5): 732-45(1996)). In addition, other CDR boundary definitions may not strictly follow one of the above systems, but will still overlap with Kabat CDRs, although they may be shortened or lengthened in light of prediction or experimental findings that a particular residue or group of residues or even the entire CDRs, do not significantly affect the antigen binding. In the present application, the Chothia numbering system is used.

In the present application, the term "antigen-binding fragment" generally refers to one or more fragments of an antibody that function to specifically bind to an antigen. The antigen-binding function of an antibody can be achieved by a full-length fragment of the antibody. The antigen-binding function of an antibody may also be achieved by: a heavy chain comprising a fragment of Fv, scFv, dsFv, Fab, Fab' or F (ab')2, or a light chain comprising a fragment of Fv, scFv, dsFv, Fab, Fab', or F(ab')2. (1) An Fab fragment, i.e., a monovalent fragment consisting of the VL, VH, CL, and CH domains; (2) an F(ab')2 fragment, a bivalent fragment comprising two Fab fragments linked by a disulfide bond at the hinge region; (3) an Fd fragment consisting of the VH and CH domains; (4) an Fv fragment consisting of the VL and VH domains of a single arm of an antibody; (5) a dAb fragment consisting of VH domains (Ward et al., (1989) Nature 341: 544-546); (6) an isolated complementarity determining region (CDR), and (7) a combination of two or more isolated CDRs which may optionally be joined by a linker. For example, monovalent single chain molecules Fv (scFv) formed by the pairing of VL and VH may also be included (see, Bird et al., (1988) Science 242: 423-426; and Huston et al., (1988) Proc.Natl.Acad.Sci. 85: 5879-5883). For example, a class of antibody VHH lacking the antibody light chain but only the heavy chain variable region can also be included (see, e.g., Kang Xiaozhen et al., Chinese Journal of Biotechnology, 2018, 34 (12): 1974-1984). The "antigen-binding portion" may also include an immunoglobulin fusion protein comprising a binding domain selected from the group consisting of: (1) a binding domain polypeptide fused to an immunoglobulin hinge region polypeptide; (2) an immunoglobulin heavy chain CH2 constant region fused to the hinge region; and (3) an immunoglobulin heavy chain CH3 constant region fused to the CH2 constant region.

In the present application, the term "monoclonal antibody" generally refers to a population of substantially homologous antibodies, i.e., the individual antibodies comprising the population are identical except for possible naturally occurring mutations that may be present in minor amounts. Monoclonal antibodies are highly specific, being directed against a single antigenic site. For example, the monoclonal antibodies can be prepared by hybridoma techniques or can be produced in bacteria, eukaryotic animal or plant cells using recombinant DNA methods, or can be derived from phage antibody libraries using the techniques, for example, described in Clackson et al., Nature, 352:624-628 (1991) and Marks et al., Mol.Biol., 222:581-597 (1991).

In the present application, the term "chimeric antibody" generally refers to an antibody in which a portion of the amino acid sequence of each heavy or light chain is homologous to the corresponding amino acid sequence in an antibody from a particular species, or belongs to a particular class, while the remaining segments of the chain are homologous to the corresponding sequence in another species. For example, the variable regions of both the light and heavy chains are derived from the variable region of an antibody of one animal species (e.g., mouse, rat, etc.), while the constant portions are homologous to antibody sequences from another species (e.g., human). For example, to obtain chimeric antibodies, non-human-derived B cells or hybridoma cells can be used to generate variable regions, while which the constant regions in combination therewith are of human origin. The variable region has the advantage of being easy to prepare and its specificity is not influenced by the origin of the constant region with which it is combined. Also, since the constant region of the chimeric antibody can be derived from human, the chimeric antibodies are less likely to elicit an immune response upon injection than using antibodies whose constant regions are of non-human origin.

In the present application, the term "humanized antibody" generally refers to a chimeric antibody that contains fewer sequences from non-human immunoglobulins, thereby reducing the immunogenicity of a xenogenous antibody when introduced into humans, while maintaining the full antigen-binding affinity and specificity of the antibody. For example, non-human binding domains can be humanized using the technical means, such as CDR transplantion (Jones et al., Nature 321:522(1986)) and variants thereof; including "reshaping", (Verhoeyen, et al., 1988 Science 239:1534-1536; Riechmann, et al., 1988 Nature 332:323-337; Tempest, et al., Bio/Technol 1991 9:266-271), "hyperchimerization" (Queen, et al., 1989 Proc Natl Acad Sci USA 86:10029-10033; Co, et al., 1991 Proc Natl Acad Sci USA 88:2869-2873; Co, et al., 1992 J Immunol 148:1149-1154), and "veneering" (Mark, et al., "Derivation of therapeutically active humanized and veneered anti-CD18 antibodies." In: Metcalf B W, Dalton B J, eds. Cellular adhesion: molecular definition to therapeutic potential. New York: Plenum Press, 1994: 291-312), and resurfacing (US5639641). Other regions, such as hinge and constant region domains, may also be humanized if they are also derived from non-human sources.

In the present application, the term "murine antibody" generally refers to an antibody in which the variable region framework and CDR regions are derived from mouse germline immunoglobulin sequences. In addition, if the antibody comprises constant regions, which are also derived from mouse germline immunoglobulin sequences. The murine antibodies of the present application may comprise amino acid residues not encoded by the mouse germline immunoglobulin sequences, such as mutations introduced by random or point mutations in vitro or by somatic mutation in vivo. However, the term "murine antibody" does not comprise the antibodies having CDR sequences from other mammalian species inserted into the framework sequences of a mouse.

In the present application, the terms "GARP protein" or "GARP antigen" are used interchangeably and include any variants and homologues of GARP. The GARP is expressed naturally by a cell or is expressed by a cell transfected with a GARP gene. In the present application, GARP can be a human GARP having an accession number Q14392 in UniProt/Swiss-Prot. In the present application, GARP can be expressed on the surface of immune cells. For example, it can be expressed on the surface of regulatory T cells (Treg).

In the present application, the terms "TGF-β1" and "TGF β1" are used interchangeably and generally associated with cell growth regulation and differentiation. In the present application, the TGF-β1 is an isoform of TGF-β. In the present application, the "TGF-β1" may include any variants and homologues of TGF-β1 that are expressed naturally by a cell or in a cell transfected with a TGF-β1 gene. In the present application, the "TGF-β1" may be human TGF-β1, which has accession number P01137 in UniProt/Swiss-Prot. In the present application, the "TGF-β1" can be activated by GARP and binds to the TGF β receptor on CD8+T cells to inhibit the killing function of CD8+T cells on tumor cells.

In addition to the specific proteins and nucleotides mentioned herein, the present application may also include functional variants, derivatives, analogs, homologs, and fragments thereof.

The term "functional variant" refers to an amino acid sequence that is substantially identical to a naturally occurring sequence or a polypeptide encoded by a substantially identical nucleotide sequence and capable of having one or more activities of the naturally occurring sequence. In the context of the present application, a variant of any given sequence refers to a sequence in which the particular sequence of residues, whether amino acid or nucleotide residues, has been modified such that the polypeptide or polynucleotide substantially retains at least one endogenous function. Variant sequences may be obtained by the addition, deletion, substitution, modification, substitution and/or variation of at least one amino acid residue and/or nucleotide residue present in a naturally occurring protein and/or polynucleotide, so long as the original functional activity is retained.

In the present application, the term "derivative" generally refers to a polypeptide or polynucleotide of the present application comprising any substitution, variation, modification, replacement, deletion and/or addition of one (or more) amino acid residues from/on the sequence, so long as the resulting polypeptide or polynucleotide substantially retains at least one of its endogenous functions.

In the present application, the term "analog" generally refers to a polypeptide or polynucleotide including any mimetic of a polypeptide or polynucleotide, i.e., a chemical compound that possesses at least one endogenous function of the polypeptide or polynucleotide that the mimetic mimics.

Generally, amino acid substitutions, e.g., at least one (e.g., 1, 2, 3, 4, 5, 6, 7, 8, 9, 10, or 20 or more) amino acid substitution can be made, so long as the modified sequence substantially retains the desired activity or ability. Amino acid substitutions can include the use of non-naturally occurring analogs.

In the present application, the term "homologue" generally refers to an amino acid sequence or nucleotide sequence having certain homology to a naturally occurring sequence. The term "homology" can be equivalent to sequence "identity". Homologous sequences may include amino acid sequences that may be at least 80%, 85%, 90%, 99.1%, 99.2%, 99.3%, 99.4%, 99.5%, 99.6%, 99.7%, 99.8%, or 99.9% identical to the subject sequence. Typically, the homologue will comprise the same active site or the like as the subject amino acid sequence. Homology may be considered in terms of similarity (i.e., amino acid residues having similar chemical properties/functions) or may be expressed in terms of sequence identity. In the present application, a sequence having percent identity in any one of the SEQ ID NO of a mentioned amino acid sequence or nucleotide sequence refers to a sequence having the percent identity over the entire length of the mentioned SEQ ID NO. To determine sequence identity, sequence alignments may be performed by a variety of ways known to those skilled in the art, e.g., using BLAST, BLAST-2, ALIGN, NEEDLE, or Megalign (DNASTAR) software, etc. Those skilled in the art can determine the appropriate parameters for the alignment, including any algorithm required to achieve the optimal alignment over the full length of the sequence being compared.

The proteins or polypeptides used in the present application may also have deletions, insertions, or substitutions of amino acid residues which produce silent changes and result in functionally equivalent proteins. Intentional amino acid substitutions may be made on the basis of similarity in polarity, charge, solubility, hydrophobicity, hydrophilicity, and/or amphipathic nature of the residues, so long as the endogenous function is retained. For example, negatively charged amino acids include aspartic acid and glutamic acid; positively charged amino acids include lysine and arginine; and amino acids containing uncharged polar head groups with similar hydrophilicity values include asparagine, glutamine, serine, threonine, and tyrosine.

In the present application, the term "tumor" generally refers to a neoplasm formed by the proliferation of local tissue cells under the action of various tumorigenic factors. For example, the tumor may include a solid tumor. For example, the tumor may include a tumor associated with protein expression of GARP. The term "tumor associated with protein expression of GARP" generally refers to a tumor in which GARP expression leads to disease progression or evades immune surveillance. The tumor associated with protein expression of GARP can be a GARP positive tumor. In GARP positive tumors, the protein expression amount of GAPR on the surface of the tumor cells is about 1%, 5%, 10%, 15%, 20%, 25%, 30%, 35%, 40%, 50%, 60%, 70%, 80% or more higher than that of normal cells. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor.

In the present application, the term "melanoma" generally refers to a highly malignant tumor of melanocytes origin. In the present application, the melanoma can occur in the skin as well as in the mucosa and viscera.

In the present application, the term "immunoconjugate" generally refers to a conjugate formed by the conjugation of other agents (e.g., a chemotherapeutic agent, a radioactive element, a cytostatic agent, and a cytotoxic agent) to the isolated antigen-binding protein (e.g., via covalent attachment of a linking molecule), wherein the conjugate can deliver the other agents to a target cell (e.g., a tumor cell) via specific binding of the isolated antigen-binding protein to an antigen on the target cell. The immunoconjugate then undergoes such internalization and eventually enters the interior of the target cell (e.g., into vesicles such as a lysosome), at which point the linker molecule in the immunoconjugate can be cleaved to release the other agent, thereby exerting its cytotoxic effect. In addition, the antigen may also be secreted by the target cell and located in the space outside the target cell.

In the present application, the term "pharmaceutically acceptable therapeutic agent" generally refers to an agent that can inhibit the proliferation of tumors and/or tumor cells. In the present application, the therapeutic agent may be a cytotoxic agent or a cytostatic agent. For example, the therapeutic agent may be selected from the group consisting of: mitotic inhibitors, kinase inhibitors, alkylating agents, antimetabolites, intercalating antibiotics, growth factor inhibitors, cell cycle inhibitors, enzymes, topoisomerase inhibitors, histone deacetylase inhibitors, anti-survival agents, and biological response modifiers.

In the present application, the term "subject" generally refers to human or non-human animals, including, but not limited to, cats, dogs, horses, pigs, cows, caprid, rabbits, mice, rats, or monkeys.

In the present application, the term "nucleic acid molecule" generally refers to an isolated form of nucleotides, deoxyribonucleotides, or ribonucleotides or analogs thereof, of any length, isolated from their natural environment or artificially synthesized.

In the present application, the term "vector" generally refers to a nucleic acid molecule capable of self-replication in a suitable host. The vector can transfer the inserted nucleic acid molecule into and/or between cells. The vector may include a vector for primarily inserting DNA or RNA into a cell, a vector for primarily replicating DNA or RNA, and a vector for primarily expressing transcription and/or translation of DNA or RNA. The vector can be a polynucleotide capable of being transcribed and translated into a polypeptide when introduced into an appropriate cell. In general, the vector may produce the desired expression product by culturing an appropriate cell containing the vector. In the present application, the vector may include a lentiviral vector.

In the present application, the term "cell" generally refers to an individual cell, cell line or cell culture that may or may already contain a plasmid or vector comprising a nucleic acid molecule as described herein, or that is capable of expressing a polypeptide as described herein or an antigen-binding protein as described herein. The cell may include the progeny of a single cell. Due to natural, accidental, or deliberate mutations, the progeny cells may not necessarily be identical in morphology or in genome to the original parent cell, but are capable of expressing the polypeptide or antigen-binding protein as described herein. The cells can be obtained by transfecting cells in vitro with the vectors as described herein. The cells may be prokaryotic cells (e.g., E. coli) or eukaryotic cells (e.g., yeast cell, COS cells, Chinese Hamster Ovary (CHO) cells, HeLa cells, HEK293 cells, COS-1 cells, NS0 cells, or myeloma cells). In some embodiments, the cells may be immune cells. For example, the immune cell may be selected from the group consisting of T cells, B cells, natural killer cells (NK cells), macrophages, NKT cells, monocytes, dendritic cells, granulocytes, lymphocytes, leukocytes and/or peripheral blood mononuclear cells. For example, the immune cells may be T cells.

In the present application, the term "treatment" generally refers to: (i) the prevention of the development of a disease, disorder, and/or condition in a patient who may be susceptible to, but has not yet been diagnosed with, that disease, disorder, or condition; (ii) the suppression of the disease, disorder, or condition, i.e., the curb of the development; and (iii) remission of the disease, disorder, or condition, i.e., causing regression of the disease, disorder, and/or condition and/or symptoms associated with the disease, disorder, and/or condition.

In the present application, the terms "polypeptide", "peptide", and "protein" are used interchangeably and generally refer to a polymer of amino acids of any length. The polymer may be linear or branched, and may comprise modified amino acids and it may be interrupted by non-amino acids. These terms also encompass amino acid polymers that have been modified. These modifications may comprise: disulfide bond formation, glycosylation, lipidation, acetylation, phosphorylation, or any other manipulation (e.g., binding to a labeling component). The term "amino acid" includes natural and/or non-natural or synthetic amino acids, including glycine as well as the D and L optical isomers, as well as amino acid analogs and peptidomimetics.

In the present application, the terms "polynucleotide", "nucleotide", "nucleotide sequence", "nucleic acid", and "oligonucleotide" are used interchangeably and refer generally to polymeric forms of nucleotides of any length, such as deoxyribonucleotides or ribonucleotides, or analogs thereof. A polynucleotide may have any three-dimensional structure and may perform any function that is known or unknown. Non-limiting examples of polynucleotides are as follows: a coding or noncoding region of a gene or gene fragment, a plurality of loci (one locus) as defined by ligation analysis, exons, introns, messenger RNA (mRNA), transport RNA, ribosomal RNA, short interfering RNA (siRNA), short hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs. If present, modification of the nucleotide structure may be performed before or after assembly of the polymer. The sequence of nucleotides may be interrupted by non-nucleotide components. The polynucleotides may be further modified after polymerization, e.g., by conjugation to labeled components.

In the present application, the term "K_{D}" (likewise, *"K_{D}"* or "KD ") generally refers to an "affinity constant" or an "equilibrium dissociation constant" and refers to a value obtained at equilibrium in a titration measurement, or by dividing the dissociation rate constant (k_{d}) by the binding rate constant (kₐ). The binding affinity of a binding protein (e.g., an isolated antigen-binding protein as described herein) for an antigen (e.g., a GARP protein) is expressed using an association rate constant (kₐ), a dissociation rate constant (k_{d}), and an equilibrium dissociation constant (K_{D}). Methods for determining association and dissociation rate constants are well known in the art. The use of fluorescence-based techniques provides high sensitivity and the ability to examine samples at equilibrium in physiological buffers. For example, the *K_{D}* value can be determined by Octet assay, and other experimental approaches and instruments such as BIAcore (Biomolecular Interaction Analysis) can be used (e.g., instruments available from BIAcoreInternationalAB, aGEHealthcarecompany, Uppsala, Sweden). Alternatively, the *K_{D}* value can be determined using KinExA (dynamic exclusion assay (KineticExclusionAssay)) available from SapidyneInstruments (Boise, Idaho) or using a surface plasmon resonance (SPR) instrument.

In the present application, the term "and/or" should be understood as meaning any one of the alternatives or both of the alternatives.

In the present application, the term "comprising " or "containing" generally refers to the inclusion of explicitly specified features, but not excluding other elements. In certain instances, "comprising" or "contain" also encompasses the inclusion of only the specified components.

In the present application, the term "about" generally refers to a range from 0.5% to 10% above or below the specified value, for example, a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the term "including" generally means comprising, summing up, encompassing or covering. In certain instances, the meaning of "being", "consisting of..." is also indicated.

### DETAILED DESCRIPTION OF THE INVENTION

### Isolated antigen-binding protein as described in the present application

In one aspect, the present application provides an isolated antigen-binding protein that can bind to a human GARP/human TGF-β1 complex with a *K_{D}* value of about 1.0E-12M or less (e.g., the *K_{D} is* no greater than about 1.0E-12M, no greater than about 0.9E-12M, no greater than about 0.8E-12M, no greater than about 0.7E-12M, no greater than about 0.6E-12M, no greater than 0.5E-12M, no greater than 0.4E-12M, no greater than 0.3E-12M, no greater than 0.2E-12M, or no greater than 0.1E-12M or less).

In the present application, the isolated antigen-binding protein may compete for binding to the human GARP/human TGF-β1 complex with a reference antibody, which may comprise a heavy chain variable region VH, which may comprise at least one, two, or three of HCDR1, HCDR2, and HCDR3.

In the present application, the HCDR3 of the reference antibody may include an amino acid sequence as set forth in SEQ ID NO: 4.

In the present application, the HCDR2 of the reference antibody may include an amino acid sequence as set forth in SEQ ID NO: 3

In the present application, the HCDR1 of the reference antibody may include an amino acid sequence as set forth in SEQ ID NO: 2.

For example, the HCDR1 of the reference antibody described herein may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4. For example, the reference antibody may include Antibody 8H2D7B3 or an antibody having the same HCDR1-3 as Antibody 8H2D7B3.

For example, the HCDR1 of the reference antibody described herein may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4. For example, the reference antibody may include Antibody JYB1907hz0 or an antibody having the same HCDR1-3 as Antibody JYB 1907hz0.

For example, the HCDR1 of the reference antibody described herein may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4. For example, the reference antibody may include Antibody JYB1907hz18 or an antibody having the same HCDR1-3 as Antibody JYB1907hz18.

For example, the VH of the reference antibody may comprise framework regions H-FR1, H-FR2, H-FR3, and H-FR4.

In the present application, the H-FR1 of the reference antibody may comprise an amino acid sequence as set forth in SEQ ID NO: 46.

QVQLX₁QSGAEX₂X₃X₄PGX₅SVKX₆SCKAS (SEQ ID NO: 46), wherein, X₁ may be Q or V, X₂ may be L or V, X₃ may be K or V, X₄ may be K or R, X₅ may be A or S, X₆ may be L or V.

In the present application, the H-FR1 of the reference antibody may comprise an amino acid sequence as set forth in SEQ ID NO: 5 or SEQ ID NO: 22.

In the present application, the H-FR2 of the reference antibody may comprise an amino acid sequence as set forth in SEQ ID NO: 47.

WMYWVX₁QX₂PX₃QGLEWIGSI (SEQ ID NO: 47), wherein X₁ may be K or R, X₂ may be A or R, and X₃ may be G or I.

In the present application, the H-FR2 of the reference antibody may comprise an amino acid sequence as set forth in SEQ ID NO: 6 or SEQ ID NO: 23.

In the present application, the H-FR3 of the reference antibody may comprise an amino acid sequence as set forth in SEQ ID NO: 48.

THYNQKFX₁X₂RX₃TVTVDKSX₄RIVYMX₅LSSLX₆SEDX₇AVYFCAR (SEQ ID NO: 48), wherein X₁ may be Q or K, X₂ may be D or G, X₃ may be A or V, X₄ may be S or T, X₅ may be E or Q, X₆ may be R or T, and X₇ may be S or T.

In the present application, the H-FR3 of the reference antibody may comprise an amino acid sequence as set forth in SEQ ID NO: 7 or SEQ ID NO: 24.

In the present application, the H-FR4 of the reference antibody may comprise an amino acid sequence as set forth in SEQ ID NO: 49.

WGX₁GTX₂VTVSS (SEQ ID NO: 49), wherein X₁ may be Q or T and X₂ may be M or T.

In the present application, the H-FR4 of the reference antibody may comprise an amino acid sequence as set forth in SEQ ID NO: 8 or SEQ ID NO: 25.

In the present application, the H-FR1 of the reference antibody may comprise an amino acid sequence as set forth in SEQ ID NO: 5 or SEQ ID NO: 22; the H-FR2 may comprise an amino acid sequence as set forth in SEQ ID NO: 6 or SEQ ID NO: 23; the H-FR3 may comprise an amino acid sequence as set forth in SEQ ID NO: 7 or SEQ ID NO: 24; and the H-FR4 may comprise an amino acid sequence as set forth in SEQ ID NO: 8 or SEQ ID NO: 25.

In the present application, the H-FR1 of the reference antibody may comprise an amino acid sequence as set forth in SEQ ID NO: 5; the H-FR2 may comprise an amino acid sequence as set forth in SEQ ID NO: 6; the H-FR3 may comprise an amino acid sequence as set forth in SEQ ID NO: 7; and the H-FR4 may comprise an amino acid sequence as set forth in SEQ ID NO: 8. For example, the reference antibody may comprise Antibody 8H2D7B3 or an antibody having the same H-FR1-4 as Antibody 8H2D7B3.

In the present application, the H-FR1 of the reference antibody may comprise an amino acid sequence as set forth in SEQ ID NO: 5; the H-FR2 may comprise an amino acid sequence as set forth in SEQ ID NO: 6; the H-FR3 may comprise an amino acid sequence as set forth in SEQ ID NO: 7; and the H-FR4 may comprise an amino acid sequence as set forth in SEQ ID NO: 8. For example, the reference antibody may include Antibody JYB1907hz0 or an antibody having the same H-FR1-4 as Antibody JYB1907hz0.

In the present application, the H-FR1 of the reference antibody may comprise an amino acid sequence as set forth in SEQ ID NO: 22; the H-FR2 may comprise an amino acid sequence as set forth in SEQ ID NO: 23; the H-FR3 may comprise an amino acid sequence as set forth in SEQ ID NO: 24; and the H-FR4 may comprise an amino acid sequence as set forth in SEQ ID NO: 25. For example, the reference antibody may include Antibody JYB1907hz18 or an antibody having the same H-FR1-4 as Antibody JYB1907hz18.

In the present application, the reference antibody may comprise a heavy chain variable region comprising an amino acid sequence as set forth in SEQ ID NO: 50.

QVQLX₁QSGAEX₂X₃X₄PGX₅SVKX₆SCKASGYTLSNYWMYWVX₇QX₈PX₉QGLEWIGSI APSDSETHYNQKFX₁₀X₁₁RX₁₂TVTVDKSX₁₃RIVYMX₁₄LSSLX₁₅SEDX₁₆AVYFCARGGFGYG SSHWYFDVWGX₁₇GTX₁₈VTVSS (SEQ ID NO: 50), wherein, X₁ may be Q or V, X₂ may be L or V, X₃ may be K or V, X₄ may be K or R, X₅ may be A or S, X₆ may be L or V, X₇ may be K or R, X₈ may be A or R, X₉ may be G or I, X₁₀ may be Q or K, X₁₁ may be D or G, X₁₂ may be A or V, X₁₃ may be S or T, X₁₄ may be E or Q, X₁₅ may be R or T, X₁₆ may be S or T, X₁₇ may be Q or T, X₁₈ may be M or T.

In the present application, the heavy chain variable region of the reference antibody may comprise an amino acid sequence as set forth in any one of SEQ ID NO: 1 and SEQ ID NO: 21.

In the present application, the reference antibody may comprise a heavy chain constant region, which may comprise an IgG-derived constant region or an IgY-derived constant region.

For example, the heavy chain constant region of the reference antibody may comprise an amino acid sequence as set forth in SEQ ID NO: 17.

In the present application, the reference antibody may comprise a light chain variable region VL, which may comprise LCDR1, LCDR2, and LCDR3.

In the present application, the HCDR3 of the reference antibody may comprise an amino acid sequence as set forth in SEQ ID NO: 12.

In the present application, the LCDR2 of the reference antibody may comprise an amino acid sequence as set forth in SEQ ID NO: 45.

GATSLEX₁ (SEQ ID NO: 45), wherein, X₁ may be S or T.

In the present application, the LCDR2 of the reference antibody may comprise an amino acid sequence as set forth in SEQ ID NO: 11 or SEQ ID NO: 28.

In the present application, the LCDR1 of the reference antibody may comprise an amino acid sequence as set forth in SEQ ID NO: 44.

X₁ASDHINKWLA (SEQ ID NO: 44), wherein, X₁ may be K or R.

In the present application, the LCDR1 of the reference antibody may comprise an amino acid sequence as set forth in SEQ ID NO: 10 or SEQ ID NO: 27.

For example, the LCDR1 of the reference antibody described herein may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the reference antibody may comprise Antibody 8H2D7B3 or an antibody having the same LCDR1-3 as Antibody 8H2D7B3.

For example, the LCDR1 of the reference antibody described herein may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the reference antibody may include Antibody JYB1907hz0 or an antibody having the same LCDR1-3 as Antibody JYB 1907hz0.

For example, the LCDR1 of the reference antibody described herein may comprise an amino acid sequence as set forth in SEQ ID NO: 27; the LCDR2 may comprise an amino acid sequence as set forth in SEQ ID NO: 28; and the LCDR3 may comprise an amino acid sequence as set forth in SEQ ID NO: 12. For example, the reference antibody may include Antibody JYB1907hz18 or an antibody having the same LCDR1-3 as Antibody JYB1907hz18.

For example, the VL of the reference antibody may comprise framework regions L-FR1, L-FR2, L-FR3, and L-FR4.

In the present application, the L-FR1 of the reference antibody may comprise an amino acid sequence as set forth in SEQ ID NO: 52.

DIQMTQSX₁X₂X₃LSX₄SX₅GX₆RVTITC (SEQ ID NO: 52), wherein, X₁ may be P or S, X₂ may be A or S, X₃ may be T or Y, X₄ may be A or V, X₅ may be L or V, X₆ may be D or G.

In the present application, the L-FR1 of the reference antibody may comprise an amino acid sequence as set forth in SEQ ID NO: 13 or SEQ ID NO: 29.

In the present application, the L-FR2 of the reference antibody may comprise an amino acid sequence as set forth in SEQ ID NO: 14

In the present application, the L-FR3 of the reference antibody may comprise an amino acid sequence as set forth in SEQ ID NO: 53.

GX₁PSRFSGSGSGKDYTLX₂IX₃X₄LQX₅X₆DX₇ATYYC (SEQ ID NO: 53), wherein, X₁ maybe I or V, X₂ may be TorS, X₃ may be TorS, X₄ may be G or S, X₅ may be P or T, X₆ may be D or E, X₇ may be F or V

In the present application, the L-FR3 of the reference antibody may comprise an amino acid sequence as set forth in SEQ ID NO: 15 or SEQ ID NO: 30.

In the present application, the L-FR4 of the reference antibody may comprise an amino acid sequence as set forth in SEQ ID NO: 54.

FGX₁GTKLEIK (SEQ ID NO: 54), wherein, X₁ may be G or Q.

In the present application, the L-FR4 of the reference antibody may comprise an amino acid sequence as set forth in SEQ ID NO: 16 or SEQ ID NO: 31.

In the present application, the L-FR1 of the reference antibody may comprise an amino acid sequence as set forth in SEQ ID NO: 13 or SEQ ID NO: 29; the L-FR2 may comprise an amino acid sequence as set forth in SEQ ID NO: 14; the L-FR3 may comprise an amino acid sequence as set forth in SEQ ID NO: 15 or SEQ ID NO: 30; and the L-FR4 may comprise an amino acid sequence as set forth in SEQ ID NO: 16 or SEQ ID NO: 31.

In the present application, the L-FR1 of the reference antibody may comprise an amino acid sequence as set forth in SEQ ID NO: 13; the L-FR2 may comprise an amino acid sequence as set forth in SEQ ID NO: 14; the L-FR3 may comprise an amino acid sequence as set forth in SEQ ID NO: 15; and the L-FR4 may comprise an amino acid sequence as set forth in SEQ ID NO: 16. For example, the reference antibody may comprise Antibody 8H2D7B3 or an antibody having the same L-FR1-4 as the antibody8H2D7B3.

In the present application, the L-FR1 of the reference antibody may comprise an amino acid sequence as set forth in SEQ ID NO: 13; the L-FR2 may comprise an amino acid sequence as set forth in SEQ ID NO: 14; the L-FR3 may comprise an amino acid sequence as set forth in SEQ ID NO: 15; and the L-FR4 may comprise an amino acid sequence as set forth in SEQ ID NO: 16. For example, the reference antibody may include Antibody JYB1907hz0 or an antibody having the same H-FR1-4 as Antibody JYB1907hz0.

In the present application, the L-FR1 of the reference antibody may comprise an amino acid sequence as set forth in SEQ ID NO: 29; the L-FR2 may comprise an amino acid sequence as set forth in SEQ ID NO: 14; the L-FR3 may comprise an amino acid sequence as set forth in SEQ ID NO: 30; and the L-FR4 may comprise an amino acid sequence as set forth in SEQ ID NO: 31. For example, the reference antibody may include Antibody JYB1907hz18 or an antibody having the same L-FR1-4 as Antibody JYB 1907hz18.

In the present application, the reference antibody may comprise a light chain variable region, which may comprise an amino acid sequence as set forth in SEQ ID NO: 51.

DIQMTQSX₁X₂X₃LSX₄SX₅GX₆RVTITCX₇ASDHINKWLAWYQQKPGNAPRLLISGATSL EX₈GX₉PSRFSGSGSGKDYTLX₁₀IX₁₁X₁₂LQX₁₃X₁₄DX₁₅ATYYCQQYWTTPYTFGX₁₆GTKLEI K (SEQ ID NO: 51), wherein, X₁ may be P or S, X₂ may be A or S, X₃ may be T or Y, X₄ may be A or V, X₅ may be L or V, X₆ may be D or G, X₇ may be K or R, X₈ may be SorT, X₉ may be I or V, X₁₀ may be T or S, X₁₁ may be T or S, X₁₂ may be G or S, X₁₃ may be P or T, X₁₄ may be D or E, X₁₅ may be F or V, X₁₆ may be G or Q.

In the present application, the light chain variable region of the reference antibody may comprise an amino acid sequence as set forth in any one of SEQ ID NO: 9 and SEQ ID NO: 26.

In the present application, the reference antibody may comprise a light chain constant region, which may comprise an Igκ-derived constant region or an Igλ-derived constant region.

For example, the reference antibody light chain constant region comprises an amino acid sequence as set forth in SEQ ID NO: 18.

In the present application, the reference antibody may comprise HCDR1-3 and LCDR1-3. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the reference antibody may comprise Antibody 8H2D7B3 or an antigen binding protein having the same HCDR1-3 and LCDR1-3 as Antibody 8H2D7B3.

In the present application, the reference antibody may comprise HCDR1-3 and LCDR1-3. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the reference antibody may include Antibody JYB1907hz0 or an antigen binding protein having the same HCDR1-3 and LCDR1-3 as Antibody JYB 1907hz0.

In the present application, the reference antibody may comprise HCDR1-3 and LCDR1-3. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 27; the LCDR2 may comprise an amino acid sequence as set forth in SEQ ID NO: 28; and the LCDR3 may comprise an amino acid sequence as set forth in SEQ ID NO: 12. For example, the reference antibody may include Antibody JYB1907hz18 or an antigen binding protein having the same HCDR1-3 and LCDR1-3 as Antibody JYB1907hz18.

In the present application, the reference antibody may comprise a heavy chain variable region and a light chain variable region, wherein the heavy chain variable region may comprise HCDR1-3 and H-FR1-4, and the light chain variable region may comprise LCDR1-3 and L-FR1-4. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the H-FR1 may comprise an amino acid sequence as set forth in SEQ ID NO: 5; the H-FR2 may comprise an amino acid sequence as set forth in SEQ ID NO: 6; the H-FR3 may comprise an amino acid sequence as set forth in SEQ ID NO: 7; and the H-FR4 may comprise an amino acid sequence as set forth in SEQ ID NO: 8; the L-FR1 may comprise an amino acid sequence as set forth in SEQ ID NO: 13; the L-FR2 may comprise an amino acid sequence as set forth in SEQ ID NO: 14; the L-FR3 may comprise an amino acid sequence as set forth in SEQ ID NO: 15; and the L-FR4 may comprise an amino acid sequence as set forth in SEQ ID NO: 16. For example, the heavy chain variable region of the reference antibody may comprise an amino acid sequence as set forth in SEQ ID NO: 1. For example, the reference antibody may comprise an antigen-binding fragment 8H2D7B3 or an antigen-binding protein having the same heavy chain variable region as the antigen-binding fragment 8H2D7B3. For example, the light chain variable region of the reference antibody may comprise an amino acid sequence as set forth in SEQ ID NO: 9. For example, the reference antibody may comprise Antibody 8H2D7B3 or an antigen-binding protein having the same light chain variable region as Antibody 8H2D7B3.

In the present application, the reference antibody comprises a heavy chain and a light chain, the heavy chain of the reference antibody may comprise an amino acid sequence as set forth in SEQ ID NO: 19. For example, the reference antibody may comprise Antibody 8H2D7B3 or an antigen-binding protein having the same heavy chain as Antibody 8H2D7B3. For example, the light chain of the reference antibody may comprise an amino acid sequence as set forth in SEQ ID NO: 20. For example, the reference antibody may include Antibody 8H2D7B3 or an antigen-binding protein having the same light chain as Antibody 8H2D7B3.

In the present application, the reference antibody may comprise a heavy chain variable region and a light chain variable region, wherein the heavy chain variable region may comprise HCDR1-3 and H-FR1-4, and the light chain variable region may comprise LCDR1-3 and L-FR1-4. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the H-FR1 may comprise an amino acid sequence as set forth in SEQ ID NO: 5; the H-FR2 may comprise an amino acid sequence as set forth in SEQ ID NO: 6; the H-FR3 may comprise an amino acid sequence as set forth in SEQ ID NO: 7; and the H-FR4 may comprise an amino acid sequence as set forth in SEQ ID NO: 8; the L-FR1 may comprise an amino acid sequence as set forth in SEQ ID NO: 13; the L-FR2 may comprise an amino acid sequence as set forth in SEQ ID NO: 14; the L-FR3 may comprise an amino acid sequence as set forth in SEQ ID NO: 15; and the L-FR4 may comprise an amino acid sequence as set forth in SEQ ID NO: 16. For example, the heavy chain variable region of the reference antibody may comprise an amino acid sequence as set forth in SEQ ID NO: 1. For example, the reference antibody may comprise an antigen-binding fragment JYB1907hz0 or an antigen-binding protein having the same heavy chain variable region as the antigen-binding fragment JYB1907hz0. For example, the light chain variable region of the reference antibody may comprise an amino acid sequence as set forth in SEQ ID NO: 9. For example, the reference antibody may include Antibody JYB 1907hz0 or an antigen-binding protein having the same light chain variable region as Antibody JYB 1907hz0.

In the present application, the reference antibody may comprise a heavy chain and a light chain, the heavy chain of the reference antibody may comprise an amino acid sequence as set forth in SEQ ID NO: 19. For example, the reference antibody may include Antibody JYB1907hz0 or an antigen-binding protein having the same heavy chain as Antibody JYB1907hz0. For example, the light chain of the reference antibody may comprise an amino acid sequence as set forth in SEQ ID NO: 20. For example, the reference antibody may include Antibody JYB1907hz0 or an antigen-binding protein having the same light chain as Antibody JYB 1907hz0.

In the present application, the reference antibody may comprise a heavy chain variable region and a light chain variable region, wherein the heavy chain variable region may comprise HCDR1-3 and H-FR1-4, and the light chain variable region may comprise LCDR1-3 and L-FR1-4. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 27; the LCDR2 may comprise an amino acid sequence as set forth in SEQ ID NO: 28; and the LCDR3 may comprise an amino acid sequence as set forth in SEQ ID NO: 12. For example, the H-FR1 may comprise an amino acid sequence as set forth in SEQ ID NO: 22; the H-FR2 may comprise an amino acid sequence as set forth in SEQ ID NO: 23; the H-FR3 may comprise an amino acid sequence as set forth in SEQ ID NO: 24; and the H-FR4 may comprise an amino acid sequence as set forth in SEQ ID NO: 25; the L-FR1 may comprise an amino acid sequence as set forth in SEQ ID NO: 29; the L-FR2 may comprise an amino acid sequence as set forth in SEQ ID NO: 14; the L-FR3 may comprise an amino acid sequence as set forth in SEQ ID NO: 30; and the L-FR4 may comprise an amino acid sequence as set forth in SEQ ID NO: 31. For example, the heavy chain variable region of the reference antibody may comprise an amino acid sequence as set forth in SEQ ID NO: 21. For example, the reference antibody may comprise an antigen-binding fragment JYB1907hz18 or an antigen-binding protein having the same heavy chain variable region as the antigen-binding fragment JYB1907hz18. For example, the light chain variable region of the reference antibody may comprise an amino acid sequence as set forth in SEQ ID NO: 26. For example, the reference antibody may include Antibody JYB1907hz18 or an antigen-binding protein having the same light chain variable region as Antibody JYB1907hz18.

In the present application, the reference antibody may comprise a heavy chain and a light chain. For example, the heavy chain of the reference antibody may comprise an amino acid sequence as set forth in SEQ ID NO: 32. For example, the reference antibody may include Antibody JYB1907hz18 or an antigen-binding protein having the same heavy chain as Antibody JYB1907hz18. For example, the light chain of the reference antibody may comprise an amino acid sequence as set forth in SEQ ID NO: 33. For example, the reference antibody may include Antibody JYB1907hz18 or an antigen-binding protein having the same light chain as Antibody JYB1907hz18.

In the present application, the isolated antigen-binding protein may bind to the human GARP/human TGF-β1 complex.

The isolated antigen-binding protein described herein may remit or treat a tumor, wherein the tumor may include a solid tumor. For example, tumors associated with the expression of GARP may be included. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor. For example, the tumor may be a melanoma, a breast cancer and/or a lung squamous carcinoma. For example, the tumor may be a melanoma.

In the present application, the isolated antigen-binding protein may comprise a heavy chain variable region VH which may comprise at least one, at least two, or at least three of HCDR1, HCDR2, and HCDR3.

In the present application, the HCDR3 of the antigen-binding protein may comprise an amino acid sequence as set forth in SEQ ID NO: 4.

In the present application, the HCDR2 of the antigen-binding protein may comprise an amino acid sequence as set forth in SEQ ID NO: 3.

In the present application, the HCDR1 of the antigen-binding protein may comprise an amino acid sequence as set forth in SEQ ID NO: 2.

For example, the HCDR1 of an isolated antigen-binding protein described herein may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4. For example, the isolated antigen-binding protein may comprise Antibody 8H2D7B3 or an antibody having the same HCDR1-3 as Antibody 8H2D7B3.

For example, the HCDR1 of an isolated antigen-binding protein described herein may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4. For example, the isolated antigen-binding protein may include Antibody JYB1907hz0 or an antibody having the same HCDR1-3 as Antibody JYB 1907hz0.

For example, the HCDR1 of an isolated antigen-binding protein described herein may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4. For example, the isolated antigen-binding protein may include Antibody JYB1907hz18 or an antibody having the same HCDR1-3 as Antibody JYB1907hz18.

For example, the VH of the antigen-binding protein may comprise framework regions H-FR1, H-FR2, H-FR3, and H-FR4.

In the present application, the H-FR1 of the antigen-binding protein may comprise an amino acid sequence as set forth in SEQ ID NO: 46.

QVQLX₁QSGAEX₂X₃X₄PGX₅SVKX₆SCKAS (SEQ ID NO: 46), wherein, X₁ may be Q or V, X₂ may be L or V, X₃ may be K or V, X₄ may be K or R, X₅ may be A or S, X₆ may be L or V

In the present application, the H-FR1 of the antigen-binding protein may comprise an amino acid sequence as set forth in SEQ ID NO: 5 or SEQ ID NO: 22.

In the present application, the H-FR2 of the antigen-binding protein may comprise an amino acid sequence as set forth in SEQ ID NO: 47.

WMYWVX₁QX₂PX₃QGLEWIGSI (SEQ ID NO: 47), wherein X₁ may be K or R, X₂ may be A or R, and X₃ may be G or I.

In the present application, the H-FR2 of the antigen-binding protein may comprise an amino acid sequence as set forth in SEQ ID NO: 6 or SEQ ID NO: 23.

In the present application, the H-FR3 of the antigen-binding protein may comprise an amino acid sequence as set forth in SEQ ID NO: 48.

THYNQKFX₁X₂RX₃TVTVDKSX₄RIVYMX₅LSSLX₆SEDX₇AVYFCAR (SEQ ID NO: 48), wherein X₁ may be Q or K, X₂ may be D or G, X₃ may be A or V, X₄ may be S or T, X₅ may be E or Q, X₆ may be R or T, and X₇ may be S or T.

In the present application, the H-FR3 of the antigen-binding protein may comprise an amino acid sequence as set forth in SEQ ID NO: 7 or SEQ ID NO: 24.

In the present application, the H-FR4 of the antigen-binding protein may comprise an amino acid sequence as set forth in SEQ ID NO: 49.

WGX₁GTX₂VTVSS (SEQ ID NO: 49), X₁ may be Q or T and X₂ may be M or T.

In the present application, the H-FR4 of the antigen-binding protein may comprise an amino acid sequence as set forth in SEQ ID NO: 8 or SEQ ID NO: 25.

In the present application, the H-FR1 of the antigen-binding protein may comprise an amino acid sequence as set forth in SEQ ID NO: 5 or SEQ ID NO: 22; the H-FR2 of the antigen-binding protein may comprise an amino acid sequence as set forth in SEQ ID NO: 6 or SEQ ID NO: 23; the H-FR3 of the antigen-binding protein may comprise an amino acid sequence as set forth in SEQ ID NO: 7 or SEQ ID NO: 24; and the H-FR4 of the antigen-binding protein may comprise an amino acid sequence as set forth in SEQ ID NO: 8 or SEQ ID NO: 25.

In the present application, the H-FR1 of the antigen-binding protein may comprise an amino acid sequence as set forth in SEQ ID NO: 5; the H-FR2 of the antigen-binding protein may comprise an amino acid sequence as set forth in SEQ ID NO: 6; the H-FR3 of the antigen-binding protein may comprise an amino acid sequence as set forth in SEQ ID NO: 7; and the H-FR4 of the antigen-binding protein may comprise an amino acid sequence as set forth in SEQ ID NO: 8. For example, the isolated antigen-binding protein may comprise Antibody 8H2D7B3 or an antibody having the same H-FR1-4 as Antibody 8H2D7B3.

In the present application, the H-FR1 of the antigen-binding protein may comprise an amino acid sequence as set forth in SEQ ID NO: 5; the H-FR2 of the antigen-binding protein may comprise an amino acid sequence as set forth in SEQ ID NO: 6; the H-FR3 of the antigen-binding protein may comprise an amino acid sequence as set forth in SEQ ID NO: 7; and the H-FR4 of the antigen-binding protein may comprise an amino acid sequence as set forth in SEQ ID NO: 8. For example, the isolated antigen-binding protein may include Antibody JYB1907hz0 or an antibody having the same H-FR1-4 as Antibody JYB 1907hz0.

In the present application, the H-FR1 of the antigen-binding protein may comprise an amino acid sequence as set forth in SEQ ID NO: 22; the H-FR2 of the antigen-binding protein may comprise an amino acid sequence as set forth in SEQ ID NO: 23; the H-FR3 of the antigen-binding protein may comprise an amino acid sequence as set forth in SEQ ID NO: 24; and the H-FR4 of the antigen-binding protein may comprise an amino acid sequence as set forth in SEQ ID NO: 25. For example, the isolated antigen-binding protein may include Antibody JYB 1907hz 18 or an antibody having the same H-FR1-4 as Antibody JYB 1907hz18.

In the present application, the isolated antigen-binding protein may comprise a heavy chain variable region, which may comprise an amino acid sequence as set forth in SEQ ID NO: 50.

QVQLX₁QSGAEX₂X₃X₄PGX₅SVKX₆SCKASGYTLSNYWMYWVX₇QX₈PX₉QGLEWIGSI APSDSETHYNQKFX₁₀X₁₁RX₁₂TVTVDKSX₁₃RIVYMX₁₄LSSLX₁₅SEDX₁₆AVYFCARGGFGYG SSHWYFDVWGX₁₇GTX₁₈VTVSS (SEQ ID NO: 50), wherein, X₁ may be Q or V, X₂ may be L or V, X₃ may be K or V, X₄ may be K or R, X₅ may be A or S, X₆ may be L or V, X₇ may be K or R, X₈ may be A or R, X₉ may be G or I, X₁₀ may be Q or K, X₁₁ may be D or G, X₁₂ may be A or V, X₁₃ may be S or T, X₁₄ may be E or Q, X₁₅ may be R or T, X₁₆ may be S or T, X₁₇ may be Q or T, X₁₈ may be M or T.

In the present application, the antigen-binding protein heavy chain variable region may comprise an amino acid sequence as set forth in SEQ ID NO: 1 or SEQ ID NO: 21.

In the present application, the isolated antigen-binding protein may comprise a heavy chain constant region, which may comprise an IgG-derived constant region or an IgY-derived constant region. For example, the heavy chain constant region may comprise an IgG1-derived or IgG4-derived constant region.

For example, the antigen-binding protein light chain constant region may comprise an amino acid sequence as set forth in SEQ ID NO: 17.

In the present application, the isolated antigen-binding protein may comprise a light chain variable region VL, which may comprise LCDR1, LCDR2, and LCDR3.

In the present application, the LCDR3 of the antigen-binding protein may comprise an amino acid sequence as set forth in SEQ ID NO: 12.

In the present application, the LCDR2 of the antigen-binding protein may comprise an amino acid sequence as set forth in SEQ ID NO: 45.

GATSLEX₁ (SEQ ID NO: 45), wherein, X₁ may be S or T.

In the present application, the LCDR2 of the antigen-binding protein may comprise an amino acid sequence as set forth in SEQ ID NO: 11 or SEQ ID NO: 28.

In the present application, the LCDR1 of the antigen-binding protein may comprise an amino acid sequence as set forth in SEQ ID NO: 44.

X₁ASDHINKWLA (SEQ ID NO: 44), wherein, X₁ may be K or R.

In the present application, the LCDR1 of the antigen-binding protein may comprise an amino acid sequence as set forth in SEQ ID NO: 10 or SEQ ID NO: 27.

For example, the LCDR1 of the antigen-binding protein may comprise an amino acid sequence as set forth in SEQ ID NO: 10; the LCDR2 of the antigen-binding protein may comprise an amino acid sequence as set forth in SEQ ID NO: 11; and the LCDR3 of the antigen-binding protein may comprise an amino acid sequence as set forth in SEQ ID NO: 12. For example, the isolated antigen-binding protein may comprise Antibody 8H2D7B3 or an antibody having the same LCDR1-3 as Antibody 8H2D7B3.

For example, the LCDR1 of the antigen-binding protein may comprise an amino acid sequence as set forth in SEQ ID NO: 10; the LCDR2 of the antigen-binding protein may comprise an amino acid sequence as set forth in SEQ ID NO: 11; and the LCDR3 of the antigen-binding protein may comprise an amino acid sequence as set forth in SEQ ID NO: 12. For example, the isolated antigen-binding protein may include Antibody JYB1907hz0 or an antibody having the same LCDR1-3 as Antibody JYB1907hz0.

For example, the LCDR1 of the antigen-binding protein may comprise an amino acid sequence as set forth in SEQ ID NO: 27; the LCDR2 of the antigen-binding protein may comprise an amino acid sequence as set forth in SEQ ID NO: 28; and the LCDR3 of the antigen-binding protein may comprise an amino acid sequence as set forth in SEQ ID NO: 12. For example, the isolated antigen-binding protein may include Antibody JYB1907hz18 or an antibody having the same LCDR1-3 as Antibody JYB1907hz18.

For example, the VL of the antigen-binding protein may comprise framework regions L-FR1, L-FR2, L-FR3, and L-FR4.

In the present application, the L-FR1 of the antigen-binding protein may comprise an amino acid sequence as set forth in SEQ ID NO: 52.

DIQMTQSX₁X₂X₃LSX₄SX₅GX₆RVTITC (SEQ ID NO: 52), wherein, X₁ may be P or S, X₂ may be A or S, X₃ may be T or Y, X₄ may be A or V, X₅ may be L or V, X₆ may be D or G.

In the present application, the L-FR1 of the antigen-binding protein may comprise an amino acid sequence as set forth in SEQ ID NO: 13 or SEQ ID NO: 29.

In the present application, the L-FR2 of the antigen-binding protein may comprise an amino acid sequence as set forth in SEQ ID NO: 14.

In the present application, the L-FR3 of the antigen-binding protein may comprise an amino acid sequence as set forth in SEQ ID NO: 53.

GX₁PSRFSGSGSGKDYTLX₂IX₃X₄LQX₅X₆DX₇ATYYC (SEQ ID NO: 53), wherein, X₁ may be I or V, X₂ may be T or S, X₃ may be T or S, X₄ may be G or S, X₅ may be P or T, X₆ may be D or E, X₇ may be F or V

In the present application, the L-FR3 of the antigen-binding protein may comprise an amino acid sequence as set forth in SEQ ID NO: 15 or SEQ ID NO: 30.

In the present application, the L-FR4 of the antigen-binding protein may comprise an amino acid sequence as set forth in SEQ ID NO: 54.

FGX₁GTKLEIK (SEQ ID NO: 54), wherein, X₁ may be G or Q.

In the present application, the L-FR4 of the antigen-binding protein may comprise an amino acid sequence as set forth in SEQ ID NO: 16 or SEQ ID NO: 31.

In the present application, the L-FR1 of the antigen-binding protein may comprise an amino acid sequence as set forth in SEQ ID NO: 13 or SEQ ID NO: 29; the L-FR2 of the antigen-binding protein may comprise an amino acid sequence as set forth in SEQ ID NO: 14; the L-FR3 of the antigen-binding protein may comprise an amino acid sequence as set forth in SEQ ID NO: 15 or SEQ ID NO: 30; and the L-FR4 of the antigen-binding protein may comprise an amino acid sequence as set forth in SEQ ID NO: 16 or SEQ ID NO: 31.

In the present application, the L-FR1 of the antigen-binding protein may comprise an amino acid sequence as set forth in SEQ ID NO: 13; the L-FR2 of the antigen-binding protein may comprise an amino acid sequence as set forth in SEQ ID NO: 14; the L-FR3 of the antigen-binding protein may comprise an amino acid sequence as set forth in SEQ ID NO: 15; and the L-FR4 of the antigen-binding protein may comprise an amino acid sequence as set forth in SEQ ID NO: 16. For example, the isolated antigen-binding protein may comprise Antibody 8H2D7B3 or an antibody having the same L-FR1-4 as Antibody 8H2D7B3.

In the present application, the L-FR1 of the antigen-binding protein may comprise an amino acid sequence as set forth in SEQ ID NO: 13; the L-FR2 of the antigen-binding protein may comprise an amino acid sequence as set forth in SEQ ID NO: 14; the L-FR3 of the antigen-binding protein may comprise an amino acid sequence as set forth in SEQ ID NO: 15; and the L-FR4 of the antigen-binding protein may comprise an amino acid sequence as set forth in SEQ ID NO: 16. For example, the isolated antigen-binding protein may include Antibody JYB1907hz0 or an antibody having the same H-FR1-4 as Antibody JYB 1907hz0.

In the present application, the L-FR1 of the antigen-binding protein may comprise an amino acid sequence as set forth in SEQ ID NO: 29; the L-FR2 of the antigen-binding protein may comprise an amino acid sequence as set forth in SEQ ID NO: 14; the L-FR3 of the antigen-binding protein may comprise an amino acid sequence as set forth in SEQ ID NO: 30; and the L-FR4 of the antigen-binding protein may comprise an amino acid sequence as set forth in SEQ ID NO: 31. For example, the isolated antigen-binding protein may include Antibody JYB1907hz18 or an antibody having the same L-FR1-4 as Antibody JYB 1907hz18.

In the present application, the isolated antigen-binding protein may comprise a light chain variable region, which may comprise an amino acid sequence as set forth in SEQ ID NO: 51.

DIQMTQSX₁X₂X₃LSX₄SX₅GX₆RVTITCX₇ASDHINKWLAWYQQKPGNAPRLLISGATSL EX₈GX₉PSRFSGSGSGKDYTLX₁₀IX₁₁X₁₂LQX₁₃X₁₄DX₁₅ATYYCQQYWTTPYTFGX₁₆GTKLEI K (SEQ ID NO: 51), wherein, X₁ may be P or S, X₂ may be A or S, X₃ may be T or Y, X₄ may be A or V, X₅ may be L or V, X₆ may be D or G, X₇ may be K or R, X₈ may be SorT, X₉ may be I or V, X₁₀ may be T or S, X₁₁ may be T or S, X₁₂ may be G or S, X₁₃ may be P or T, X₁₄ may be D or E, X₁₅ may be F or V, X₁₆ may be G or Q.

In the present application, the antigen-binding protein light chain variable region may comprise an amino acid sequence as set forth in any one of SEQ ID NO: 9 and SEQ ID NO: 26.

In the present application, the isolated antigen-binding protein may comprise a light chain constant region, which comprises an Igκ-derived constant region or an Igλ-derived constant region.

For example, the antigen-binding protein light chain constant region comprises an amino acid sequence as set forth in SEQ ID NO: 18.

In the present application, the isolated antigen-binding protein may comprise HCDR1-3 and LCDR1-3. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the isolated antigen-binding protein may comprise Antibody 8H2D7B3 or an antigen-binding protein having the same HCDR1-3 and LCDR1-3 as Antibody 8H2D7B3.

In the present application, the isolated antigen-binding protein may comprise HCDR1-3 and LCDR1-3. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the isolated antigen-binding protein may include Antibody JYB1907hz0 or an antigen-binding protein having the same HCDR1-3 and LCDR1-3 as Antibody JYB1907hz0.

In the present application, the isolated antigen-binding protein may comprise HCDR1-3 and LCDR1-3. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 27; the LCDR2 may comprise an amino acid sequence as set forth in SEQ ID NO: 28; and the LCDR3 may comprise an amino acid sequence as set forth in SEQ ID NO: 12. For example, the isolated antigen-binding protein may include Antibody JYB1907hz18 or an antigen-binding protein having the same HCDR1-3 and LCDR1-3 as Antibody JYB1907hz18.

In the present application, the isolated antigen-binding protein may comprise a heavy chain variable region and a light chain variable region, wherein the heavy chain variable region may comprise HCDR1-3 and H-FR1-4, and the light chain variable region may comprise LCDR1-3 and L-FR1-4. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the H-FR1 may comprise an amino acid sequence as set forth in SEQ ID NO: 5; the H-FR2 may comprise an amino acid sequence as set forth in SEQ ID NO: 6; the H-FR3 may comprise an amino acid sequence as set forth in SEQ ID NO: 7; and the H-FR4 may comprise an amino acid sequence as set forth in SEQ ID NO: 8; the L-FR1 may comprise an amino acid sequence as set forth in SEQ ID NO: 13; the L-FR2 may comprise an amino acid sequence as set forth in SEQ ID NO: 14; the L-FR3 may comprise an amino acid sequence as set forth in SEQ ID NO: 15; and the L-FR4 may comprise an amino acid sequence as set forth in SEQ ID NO: 16. For example, the isolated antigen-binding fragment heavy chain variable region may comprise an amino acid sequence as set forth in SEQ ID NO: 1. For example, the isolated antigen-binding protein may comprise an antigen-binding fragment 8H2D7B3 or an antigen-binding protein having the same heavy chain variable region as the antigen-binding fragment 8H2D7B3. For example, the isolated antigen-binding protein light chain variable region may comprise an amino acid sequence as set forth in SEQ ID NO: 9. For example, the isolated antigen-binding protein may comprise Antibody 8H2D7B3 or an antigen-binding protein having the same light chain variable region as Antibody 8H2D7B3.

In the present application, the isolated antigen-binding protein may comprise a heavy chain and a light chain. For example, the heavy chain of the isolated antigen-binding protein may comprise an amino acid sequence as set forth in SEQ ID NO: 19. For example, the isolated antigen-binding protein may comprise Antibody 8H2D7B3 or an antigen-binding protein having the same heavy chain as Antibody 8H2D7B3. For example, the light chain of the isolated antigen-binding protein may comprise an amino acid sequence as set forth in SEQ ID NO: 20. For example, the isolated antigen-binding protein may comprise Antibody 8H2D7B3 or an antigen-binding protein having the same light chain as Antibody 8H2D7B3.

In the present application, the isolated antigen-binding protein may comprise a heavy chain variable region and a light chain variable region, wherein the heavy chain variable region may comprise HCDR1-3 and H-FR1-4, and the light chain variable region may comprise LCDR1-3 and L-FR1-4. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 10; the LCDR2 may comprise an amino acid sequence as set forth in SEQ ID NO: 11; and the LCDR3 may comprise an amino acid sequence as set forth in SEQ ID NO: 12. For example, the H-FR1 may comprise an amino acid sequence as set forth in SEQ ID NO: 5; the H-FR2 may comprise an amino acid sequence as set forth in SEQ ID NO: 6; the H-FR3 may comprise an amino acid sequence as set forth in SEQ ID NO: 7; and the H-FR4 may comprise an amino acid sequence as set forth in SEQ ID NO: 8; the L-FR1 may comprise an amino acid sequence as set forth in SEQ ID NO: 13; the L-FR2 may comprise an amino acid sequence as set forth in SEQ ID NO: 14; the L-FR3 may comprise an amino acid sequence as set forth in SEQ ID NO: 15; and the L-FR4 may comprise an amino acid sequence as set forth in SEQ ID NO: 16. For example, the heavy chain variable region of the isolated antigen-binding protein may comprise an amino acid sequence as set forth in SEQ ID NO: 1. For example, the isolated antigen-binding protein may comprise an antigen-binding fragment JYB1907hz0 or an antigen-binding protein having the same heavy chain variable region as the antigen-binding fragment JYB1907hz0. For example, the isolated antigen-binding protein light chain variable region may comprise an amino acid sequence as set forth in SEQ ID NO: 9. For example, the isolated antigen-binding protein may include Antibody JYB1907hz0 or an antigen-binding protein having the same light chain variable region as Antibody JYB 1907hz0.

In the present application, the isolated antigen-binding protein may comprise a heavy chain and a light chain. For example, the heavy chain of the isolated antigen-binding protein may comprise an amino acid sequence as set forth in SEQ ID NO: 19. For example, the isolated antigen-binding protein may include Antibody JYB1907hz0 or an antigen-binding protein having the same heavy chain as Antibody JYB1907hz0. For example, the light chain of the isolated antigen-binding protein may comprise an amino acid sequence as set forth in SEQ ID NO: 20. For example, the isolated antigen-binding protein may include Antibody JYB1907hz0 or an antigen-binding protein having the same light chain as Antibody JYB 1907hz0.

In the present application, the isolated antigen-binding protein may comprise a heavy chain variable region and a light chain variable region, wherein the heavy chain variable region may comprise HCDR1-3 and H-FR1-4, and the light chain variable region may comprise LCDR1-3 and L-FR1-4. For example, the HCDR1 may comprise an amino acid sequence as set forth in SEQ ID NO: 2; the HCDR2 may comprise an amino acid sequence as set forth in SEQ ID NO: 3; and the HCDR3 may comprise an amino acid sequence as set forth in SEQ ID NO: 4; the LCDR1 may comprise an amino acid sequence as set forth in SEQ ID NO: 27; the LCDR2 may comprise an amino acid sequence as set forth in SEQ ID NO: 28; and the LCDR3 may comprise an amino acid sequence as set forth in SEQ ID NO: 12. For example, the H-FR1 may comprise an amino acid sequence as set forth in SEQ ID NO: 22; the H-FR2 may comprise an amino acid sequence as set forth in SEQ ID NO: 23; the H-FR3 may comprise an amino acid sequence as set forth in SEQ ID NO: 24; and the H-FR4 may comprise an amino acid sequence as set forth in SEQ ID NO: 25; the L-FR1 may comprise an amino acid sequence as set forth in SEQ ID NO: 29; the L-FR2 may comprise an amino acid sequence as set forth in SEQ ID NO: 14; the L-FR3 may comprise an amino acid sequence as set forth in SEQ ID NO: 30; and the L-FR4 may comprise an amino acid sequence as set forth in SEQ ID NO: 31. For example, the isolated antigen-binding protein heavy chain variable region may comprise an amino acid sequence as set forth in SEQ ID NO: 21. For example, the isolated antigen-binding protein may include an antigen-binding fragment JYB1907hz18 or an antigen-binding protein having the same heavy chain variable region as the antigen-binding fragment JYB1907hz18. For example, the isolated antigen-binding protein light chain variable region may comprise an amino acid sequence as set forth in SEQ ID NO: 26. For example, the isolated antigen-binding protein may include Antibody JYB1907hz18 or an antigen-binding protein having the same light chain variable region as Antibody JYB1907hz18.

In the present application, the isolated antigen-binding protein may comprise a heavy chain and a light chain. For example, the heavy chain of the isolated antigen-binding protein may comprise an amino acid sequence as set forth in SEQ ID NO: 32. For example, the isolated antigen-binding protein may include Antibody JYB1907hz18 or an antigen-binding protein having the same heavy chain as Antibody JYB1907hz18. For example, the light chain of the isolated antigen-binding protein may comprise an amino acid sequence as set forth in SEQ ID NO: 33. For example, the isolated antigen-binding protein may include Antibody JYB 1907hz 18 or an antigen-binding protein having the same light chain as Antibody JYB1907hz18.

### Polypeptide and immunoconjugate

In another aspect, the present application provides one or more polypeptides, which may comprise an isolated antigen-binding protein of the present application.

In another aspect, the present application provides one or more immunoconjugates, which may comprise an isolated antigen-binding protein of the present application. In some embodiments, the immunoconjugate also comprises a pharmaceutically acceptable therapeutic agent.

In the present application, the therapeutic agent may be a cytotoxic agent or a cytostatic agent. For example, the therapeutic agent may be selected from the group consisting of: mitotic inhibitors, kinase inhibitors, alkylating agents, antimetabolites, intercalating antibiotics, growth factor inhibitors, cell cycle inhibitors, enzymes, topoisomerase inhibitors, histone deacetylase inhibitors, anti-survival agents, and biological response modifiers.

### Nucleic acid, vector, and cell

In another aspect, the present application also provides one or more isolated nucleic acid molecules that may encode the isolated antigen-binding proteins described herein. For example, each of the one or more nucleic acid molecules may encode the entire antigen-binding protein or a portion thereof (e.g., one or more of the HCDR1-3 and the heavy chain variable regions).

The nucleic acid molecules described herein may be isolated. For example, it may be produced or synthesized by the following methods: (i) in vitro amplification, e.g., by polymerase chain reaction (PCR) amplification, (ii) clonal recombination, (iii) purification, e.g., by digestion and gel electrophoresis fractionation, or (iv) synthesis, e.g., by chemical synthesis. For example, the isolated nucleic acid may be a nucleic acid molecule prepared by recombinant DNA technology.

In the present application, the nucleic acids encoding the isolated antigen-binding proteins may be prepared by a variety of methods known in the art including, but not limited to, using reverse transcription PCR and PCR to obtain nucleic acid molecules of the isolated antigen-binding proteins described herein.

In another aspect, the present application provides one or more vectors comprising one or more nucleic acid molecules described herein. Each vector may comprise one or more of the nucleic acid molecules. In addition, the vector may also comprise other genes, such as marker genes that allow for selection of the vector in an appropriate host cell and under appropriate conditions. In addition, the vector may also comprise expression control elements that allow for the proper expression of the coding region in an appropriate host. Such control elements are well known to those skilled in the art and may include, for example, promoters, ribosome binding sites, enhancers, and other control elements that regulate gene transcription or mRNA translation, etc. In some embodiments, the expression control sequence is a regulatable element. The specific structure of the expression control sequences may vary depending on the function of the species or cell type, but typically comprise 5' non-transcribed sequences and 5' and 3' non-translated sequences involved in transcription and translation initiation, respectively, such as TATA cassettes, capping sequences, CAAT sequences, etc. For example, the 5' non-transcribed expression control sequence may comprise a promoter region, which may comprise a promoter sequence for transcription control of a functionally linked nucleic acid. The expression control sequences may also include enhancer sequences or upstream activator sequences. In the present application, suitable promoters may include, for example, promoters for SP6, T3, and T7 polymerases, human U6RNA promoters, CMV promoters, and artificial hybrid promoters thereof (e.g., CMV), wherein some portion of the promoter may be fused to some portion of the promoter of a gene for another cellular proteins (e.g., human GAPDH, glyceraldehyde-3-phosphate dehydrogenase), which may or may not comprise an additional intron. One or more nucleic acid molecules described herein can be operably linked to the expression control elements.

Such vectors may include, for example, plasmids, cosmids, viruses, phages, or other vectors commonly used in, for example, genetic engineering. For example, the vector may be an expression vector. For example, the vector may be a viral vector. The patient may be administered directly (in vivo) with the viral vector or may be administered indirectly, e.g., the patient may be administered with the cell treated with the virus in vitro (ex vivo). Viral vector technology is well known in the art and is described, for example, in Sambrook et al., (2001, Molecular Cloning: A Laboratory Manual, Cold Spring Harbor Laboratory, New York) and other virology and molecular biology manuals. Conventional virus-based systems may include retroviral vectors, lentiviral vectors, adenoviral vectors, adeno-associated viral vectors, and herpes simplex viral vectors for gene transfer. In some cases, retroviral, lentiviral, and adeno-associated viral methods can be used to transfer and integrate gene into the host genome for long term expression of the inserted gene. Lentiviral vectors are retroviral vectors capable of transducing or infecting non-dividing cells and typically producing higher viral titers. Lentiviral vectors may comprise a long terminal repeat 5' LTR and a truncated 3' LTR, a RRE, a rev response element (cPPT), a central termination sequence (CTS), and/or a post-translational regulatory element (WPRE). The vectors described herein can be introduced into cells.

According to another aspect, the present application provides a cell. The cell may comprise an isolated antigen-binding protein as described herein, a polypeptide as described herein, an immunoconjugate as described herein, one or more nucleic acid molecules and/or one or more vectors as described herein. For example, each or every cell may comprise a nucleic acid molecule or vector described herein. For example, each or every cell may comprise many (e.g., 2 or more) or multiple (e.g., 2 or more) kinds of nucleic acid molecules or vectors described herein. For example, the vectors described herein can be introduced into said host cells, such as prokaryotic cells (e.g., bacterial cells), CHO cells, NS/0 cells, HEK293T cells, 293F cells, or HEK293A cells, or other eukaryotic cells, such as cells from plants, fungal or yeast cells, etc. The vectors described herein can be introduced into the host cells by methods known in the art, such as electroporation, lipofectine transfection, lipofectamin transfection, etc. For example, the cells may include yeast cells. For example, the cells may include E. coli cells. For example, the cells may include mammalian cells. For example, the cells may include immune cells.

The cells may include immune cells. In some cases, the cells may include immune cells. For example, the cells may include T cells, B cells, natural killer cells (NK cells), macrophages, NKT cells, monocytes, dendritic cells, granulocytes, lymphocytes, leukocytes and/or peripheral blood mononuclear cells.

### Pharmaceutical composition and pharmaceutical combination

In another aspect, the present application provides a pharmaceutical composition. The pharmaceutical composition may comprise the isolated antigen-binding protein, the polypeptide, the immunoconjugate, the isolated nucleic acid molecule, the vector, the cell, and/or the pharmaceutically acceptable adjuvant and/or excipient as described herein. In the present application, the pharmaceutically acceptable adjuvants may include buffers, antioxidants, preservatives, low molecular weight polypeptides, proteins, hydrophilic polymers, amino acids, sugars, chelating agents, counter ions, metal complexes and/or nonionic surfactants. Any conventional media or agent is contemplated for the pharmaceutical compositions of the present application, unless they are incompatible with the cells described herein. In the present application, the pharmaceutically acceptable excipients may include an additive other than the main drug in the pharmaceutical preparation, which may also be referred to as pharmaceutical necessities. For example, the excipients may include binders, fillers, disintegrants, and lubricants in tablets. For example, the excipients may include alcohol, vinegar, medicinal juice, etc. in traditional Chinese medicine pills. For example, the excipients may include the base portion of semi-solid formulation ointments and creams. For example, the excipients may include preservatives, antioxidants, flavoring agents, perfuming agents, solubilizing assistant, emulsifiers, solubilizers, osmotic pressure regulators, and colorants in liquid formulations.

In another aspect, the present application provides a pharmaceutical combination comprising the isolated antigen-binding protein and an immune checkpoint inhibitor.

In the present application, the immune checkpoint inhibitor may include a substance that inhibits the interaction of PD-1/PD-L1. For example, the immune checkpoint inhibitor is selected from the group consisting of: PD-1/PD-L1 blocking agents, PD-1 antagonists, PD-L1 antagonists, PD-1 inhibitors, and PD-L1 inhibitors.

For example, the PD-1/PD-L1 blocking agent may be selected from the group consisting of: BMS202 (PD-1/PD-L1 inhibitor 2), BMS-1 (PD-1/PD-L1 inhibitor 1), PD-1/PD-L1 inhibitor 3, BMS-1166 and BMS-1001.

For example, the PD-1 inhibitor may include an anti-PD-1 antibody. For example, the PD-L1 inhibitor may include a PD-L1 antibody.

For example, the anti-PD-1 antibody may be selected from the group consisting of: Nivolumab, Pembrolizumab, Camrelizumab, Toripalimab, Sintilimab, and Tislelizumab. For example, the anti-PD-L1 antibody may be selected from the group consisting of: Durvalumab, Atezolizumab, and avelumab.

In the present application, the anti-PD-L1 antibody may comprise HCDR3 of an antibody selected from the group consisting of: Durvalumab, Atezolizumab, and avelumab.

In the present application, the anti-PD-L1 antibody may comprise HCDR2 of an antibody selected from the group consisting of: Durvalumab, Atezolizumab, and avelumab.

In the present application, the anti-PD-L1 antibody may comprise HCDR1 of an antibody selected from the group consisting of: Durvalumab, Atezolizumab, and avelumab.

In the present application, the anti-PD-L1 antibody may comprise LCDR3 of an antibody selected from the group consisting of: Durvalumab, Atezolizumab, and avelumab.

In the present application, the anti-PD-L1 antibody may comprise LCDR2 of an antibody selected from the group consisting of: Durvalumab, Atezolizumab, and avelumab.

In the present application, the anti-PD-L1 antibody may comprise LCDR1 of an antibody selected from the group consisting of: Durvalumab, Atezolizumab, and avelumab.

In the present application, the anti-PD-L1 antibody may comprise a VH of an antibody selected from the group consisting of: Durvalumab, Atezolizumab, and avelumab.

In the present application, the anti-PD-L1 antibody may comprise a VL of an antibody selected from the group consisting of: Durvalumab, Atezolizumab, and avelumab. In the present application, the anti-PD-L1 antibody may comprise HCDR3, which may comprise an amino acid sequence as set forth in SEQ ID NO: 37.

In the present application, the anti-PD-L1 antibody comprises HCDR2 comprising an amino acid sequence as set forth in SEQ ID NO: 36.

In the present application, the anti-PD-L1 antibody comprises HCDR1 comprising an amino acid sequence as set forth in SEQ ID NO: 35.

In the present application, the anti-PD-L1 antibody comprises a heavy chain variable region VH which comprises HCDR1 comprising an amino acid sequence as set forth in SEQ ID NO: 35, HCDR2 comprising an amino acid sequence as set forth in SEQ ID NO: 36, and HCDR3 comprising an amino acid sequence as set forth in SEQ ID NO: 37. For example, the antibody may include Atezolizumab or an antibody having the same HCDR1-3 as the Atezolizumab.

In the present application, the anti-PD-L1 antibody may comprise a heavy chain variable region VH, which may comprise an amino acid sequence as set forth in SEQ ID NO: 34.

In the present application, the anti-PD-L1 antibody may comprise LCDR3, which may comprise an amino acid sequence as set forth in SEQ ID NO: 41.

In the present application, the anti-PD-L1 antibody may comprise LCDR2, which may comprise an amino acid sequence as set forth in SEQ ID NO: 40.

In the present application, the anti-PD-L1 antibody may comprise LCDR1, which may comprise an amino acid sequence as set forth in SEQ ID NO: 39.

In the present application, the anti-PD-L1 antibody may comprise a light chain variable region VL, which may comprise LCDR1, LCDR2, and LCDR3, wherein the LCDR3 may comprise an amino acid sequence as set forth in SEQ ID NO: 41; the LCDR2 may comprise an amino acid sequence as set forth in SEQ ID NO: 40; and the LCDR1 may comprise an amino acid sequence as set forth in SEQ ID NO: 39. For example, the antibody may include Atezolizumab or an antibody having the same LCDR1-3 as the Atezolizumab.

In the present application, the anti-PD-L1 antibody may comprise a light chain variable region VL, which may comprise an amino acid sequence as set forth in SEQ ID NO: 38.

In the present application, the anti-PD-L1 antibody may comprise a heavy chain and a light chain, wherein the heavy chain may comprise HCDR1-3 and H-FR1-4, and the light chain may comprise LCDR1-3 and L-FR1-4. For example, the HCDR1 may comprise an amino acid sequence as set forth in SEQ ID NO: 35; the HCDR2 may comprise an amino acid sequence as set forth in SEQ ID NO: 36; and the HCDR3 may comprise an amino acid sequence as set forth in SEQ ID NO: 37; the LCDR1 may comprise an amino acid sequence as set forth in SEQ ID NO: 39; the LCDR2 may comprise an amino acid sequence as set forth in SEQ ID NO: 40; and the LCDR3 may comprise an amino acid sequence as set forth in SEQ ID NO: 41. For example, the anti-PD-L1 antibody may include Atezolizumab or an antigen-binding protein having the same HCDR1-3 and LCDR1-3 as the Atezolizumab. For example, the anti-PD-L1 antibody heavy chain variable region can comprise an amino acid sequence as set forth in SEQ ID NO: 34. For example, the anti-PD-L1 antibody may include Atezolizumab or an antigen-binding protein having the same heavy chain variable region as the Atezolizumab. For example, the light chain variable region of the anti-PD-L1 antibody may comprise an amino acid sequence as set forth in SEQ ID NO: 38. For example, the anti-PD-L1 antibody may include Atezolizumab or an antigen-binding protein having the same light chain variable region as the Atezolizumab. For example, the heavy chain of the anti-PD-L1 antibody may comprise an amino acid sequence as set forth in SEQ ID NO: 42. For example, the anti-PD-L1 antibody may include Atezolizumab or an antigen-binding protein having the same heavy chain as the Atezolizumab. For example, the light chain of the anti-PD-L1 antibody may comprise an amino acid sequence as set forth in SEQ ID NO: 43. For example, the anti-PD-L1 antibody may include Atezolizumab or an antigen-binding protein having the same light chain as the Atezolizumab.

### Kit, use and method

In another aspect, the present application provides a kit comprising the pharmaceutical combination as described herein.

In another aspect, the present application provides the isolated antigen-binding protein, the polypeptide, the immunoconjugate, the isolated nucleic acid molecule, the vector, and the pharmaceutical composition for the prevention, remission, and/or treatment of a tumor.

For example, the tumor may include a solid tumor. For example, the tumor may include a tumor associated with protein expression of GARP. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor.

In another aspect, in the present application, the kit and/or the pharmaceutical combination is used for the prevention, remission, and/or treatment of a tumor.

For example, the tumor may include a solid tumor. For example, the tumor may include a tumor associated with protein expression of GARP. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor.

In another aspect, the present application provides a use of the isolated antigen-binding protein, the polypeptide, the immunoconjugate, the isolated nucleic acid molecule, the vector, the cell, and/or the pharmaceutical composition in the manufacture of a medicament for the prevention, remission, and/or treatment of a tumor.

For example, the tumor may include a solid tumor. For example, the tumor may include a tumor associated with protein expression of GARP. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor.

In another aspect, the present application provides a use of a pharmaceutical combination and/or a kit in the manufacture of a medicament for the prevention, remission, and/or treatment of a tumor.

For example, the tumor may include a solid tumor. For example, the tumor may include a tumor associated with protein expression of GARP. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor.

In another aspect, the present application provides a method for the prevention and/or treatment of a disease or disorder comprising administering to a subject in need thereof the isolated antigen-binding protein, the isolated nucleic acid molecule, the vector, the cell, and the pharmaceutical composition, wherein the disease or disorder includes a tumor.

In another aspect, the present application provides a method for the prevention and/or treatment of a disease or disorder comprising administering to a subject in need thereof the pharmaceutical combination, wherein the disease or disorder includes a tumor.

For example, the tumor may include a solid tumor. For example, the tumor may include a tumor associated with protein expression of GARP. For example, the tumor may be a metastatic colon cancer. For example, the tumor may be a hepatocellular carcinoma. For example, the tumor may be an advanced renal cell carcinoma. For example, the tumor may be a non-small cell lung cancer. For example, the tumor may be a melanoma, a breast tumor, and/or a lung tumor.

The pharmaceutical composition, pharmaceutical combination, and method described herein can be used in conjunction with other types of cancer therapy, such as chemotherapy, surgery, radiation, and gene therapy, etc. The pharmaceutical composition and method described herein can be used for other disease conditions that depend on an immune response, such as inflammation, immune diseases, and infectious diseases.

In the present application, the subject may include a human or non-human animal. For example, the non-human animal may be selected from the group consisting of: a monkey, a chicken, a goose, a cat, a dog, a mouse, and a rat. In addition, a non-human animal may also include any animal species other than human, such as livestock animals, or rodents, or primates, or domestic animals, or poultry animals. The human can be Caucasian, African, Asian, Sumerian, or other ethnicity, or a hybrid of various ethnicities. As another example, the human may be the elderly, adults, adolescents, children or infants.

Effective amounts in human can be extrapolated from the effective amounts in experimental animals. For example, Freireich et al. described the interrelationship of dosages for animals and humans (based on milligrams per meter squared of body surface) (Freireich et al., Cancer Chemother. Rep. 50, 219 (1966)). Body surface area can be approximately determined from the height and weight of the patient. See, e.g., Scientific Tables, Geigy Pharmaceuticals, Ardsley, N.Y, 537 (1970).

Without intending to be bound by any theory, the following examples are intended merely to illustrate the fusion proteins, methods of preparation, uses, etc., of the present application and are not intended to limit the scope of the present application.

### EXAMPLES

### Example 1 Preparation of recombinant plasmid and construction of immune cell strain

The full-length amino acid sequence of human GARP (designated as hGARP) (Uniprot#Q14392) and the full-length amino acid sequence of human Latent TGF-β (designated as hTGF-β1) (Uniprot#P01137) were codon-optimized to synthesize DNA sequences, which were cloned into the vectors pLVX-IRES.puro and pLVX-IRES.G418, respectively, according to the established molecular biological methods; the constructed recombinant plasmids were packaged by lentivirus, then infected into 293F cells, and screened by antibiotic resistance; the 293F-hGARP/hTGF-β1 monoclonal recombinant cell strain over-expressing hGARP/hTGF-β1 was obtained by picking a monoclonal colony, stained with flow anti-human GARP antibody (Enzo life science, ALX-804-867FI-0100) and anti-human TGF-β1 antibody (R&D Systems, FAB2463A), and loaded on a flow cytometer for detection. The results were shown in Table 1. The 293F-hGARP/hTGF-β1 monoclonal recombinant cell strain with high expression were picked for immunization. Similar methods were used for the preparation of CHOK1-hGARP/hTGF-β1, 293T-hGARP, and 293T-hTGF-β1 monoclonal recombinant cell stains for the screening and identification of monoclonal antibodies.

**Table 1 Flow cytometry assay results of 293F-hGARP/hTGF-β1 monoclonal recombinant cell strain**

| Clone Number | Mean maximum fluorescence intensity (MFI) | | | |
|---|---|---|---|---|
| | Isotype control-FITC | Anti-human GARP antibody-FITC | Isotype control-APC | Anti-LAP (TGF-β1) Antibody-APC |
| 2F4 | 101 | 8368 | 90 | 149969 |
| 2D3 | 103 | 6963 | 103 | 141919 |

### Example 2 Preparation of murine anti-human GARP monoclonal antibody

### 2.1 Immunization

Balb/c and SJL mice were immunized with 293F-hGARP/hTGF-β1 monoclonal recombinant cell strains 2F4 and 2D3 for 3-4 times, respectively, 3-5×10⁶ cells/mouse were injected at 2-3 weeks intervals, and blood was collected on the seventh day after the third immunization as post-immunization serum. The binding degree of the immunized serum was detected with CHOK1-hGARP/hTGF-β1 and blank cell CHOK1 in flow cytometry, the ratio of the average fluorescence intensity for binding to the CHOK1-hGARP/hTGF-β1 and the blank cell CHOK1 was calculated in the flow cytometry, and the mice with higher ratio were used for fusion. Three days prior to fusion, mice were subjected to booster immunization with 293F-hGARP/hTGF-β1 monoclonal recombinant cell strain at 3-5 ×10⁶ cells/mouse.

### 2.2 Fusion

On the day of fusion, after euthanasia of mice, the mice were dissected, the spleens were fetched and ground to harvest cells, which were collected by centrifugation at 1500 rpm and 5 min. The cells were suspended with 5 ml of red blood cell lysate, leaving standing at 4°C for 5 min, reactions were terminated with DMEM+10% FBS and the cells were counted. After centrifugation, the cells were suspended with 40 ml DMEM. After standing for 2-3 min, the supernatant was transferred to another 50 mL centrifuge tube. SP2/0 cells were collected, mixed with the spleen cells at a ratio of SP2/0: spleen cells=1: 5, the mixture was centrifuged, the supernatant was pipetted thoroughly, the centrifuge tube was flapped to mix the cell pellet, the mixed cells were washed twice with DMEM, and then subjected to PEG fusion or electrofusion according to the conventional method, the cells were washed with DMEM medium and resuspended in the selection medium of DMEM+10%FBS+1×HAT. The fused cells were added into a 96-well cell culture plate and cultured in a 37°C, 75% humidity, 5% CO₂ incubator for 9-10 days.

### 2.3 Screening and subcloning, purification of antibodies

Hybridoma supernatants from the 96-well cell culture plate were assayed for binding activity to CHOK1-hGARP/hTGF-β1 cells by flow cytometry.

The CHOK1-hGARP/hTGF-β1 monoclonal recombinant cell strain was expanded to 90% confluency in T-75 cell culture flasks, the culture medium was pipetted thoroughly, the culture was washed twice with PBS buffer (available from Invitrogen), then treated with enzyme-free cell dissociation buffer (Versene solution, 15040066, available from Life technology), and cells were collected. The cells were washed twice with PBS buffer, after cell counting, the cells were diluted to 2×10⁶ cells/mL with PBS buffer, FACS buffer (PBS containing 2% FBS) was added, the mixture was incubated for 15 min at room temperature, and then centrifuged and washed twice with PBS buffer. The collected cells were suspended to 3×10⁶ cells/mL with FACS buffer. The cell suspension was added to a 96 FACS reaction plate at 100 µL/well, the hybridoma supernatant from the 96-well cell culture plate was added at 100 µL/well, and the mixture was incubated at 4°C for 1 h. After centrifugation, the supernatant was discarded, a fluorescently (Alexa 488) labeled secondary antibody (avaiable from Invitrogen) was added at 100 µL/well, and the mixture was incubated for 1 h at 4°C. The cells were centrifuged and washed 3 times with FACS buffer, suspended with the addition of fixative [4% (v/v) paraformaldehyde] at 100 µL/well, after 10 minutes, the cells were centrifuged and washed 2 times with FACS buffer. The cells were suspended with 30 µL of FACS buffer and the results were detected and analyzed with a flow cytometer intellycite plus (available from Sartorius).

The cell clone corresponding to the positive hybridoma supernatant was transferred to a 24-well cell culture plate for further cultivation for 2-4 days, and the hybridoma supernatant (corresponding cell clone name: 8H2) in the 24-well cell culture plate was used to assay binding activity to CHOK1-hGARP/hTGF-β1 cells, and the results were shown in Table 2 below. The positive cells were picked from the 24-well cell culture plate to perform subcloning and subcloning screening until obtaining a stable cell strain 8H2D7B3 capable of secreting a hybridoma monoclonal antibody which can binding to CHOK1-hGARP/hTGF-β1, then the cell strain was cultured in a conventional serum-free medium for 50 ml of small-scale productiong, and purified with a conventional protein A column to obtain a purified monoclonal antibody for subsequent identification.

**Table 2 Detection results of the binding activity of hybridoma supernatant to CHOK1-hGARP/hTGF-β1 cells**

| Clone Name | Mean Fluorescence Intensity/CHOK1-hGARP/hTGF-β1 |
|---|---|
| 8H2 | 2418591.5 |

### 2.4 Determination of light and heavy chain variable region amino acid sequences

5×10⁷ hybridoma cells 8H2D7B3 were collected by centrifugation and total RNA was extracted by a Trizol method. The cDNA obtained after the reverse transcription reaction was subjected to a G-addition reaction by terminal transferase, and the DNA containing the variable region sequence was amplified with VH, VK primers, and polyC primers, and T-A cloning was performed, followed by sequencing and antibody sequence analysis.

### Example 3 Identification of murine anti-human GARP monoclonal antibody

### 3.1 Flow cytometry identification of the binding function of murine anti-human GARP monoclonal antibodies to 293F cells, 293F-hGARP cells, 293F-hTGF-β1 cells, and 293F-hGARP/hTGF-β1 complex

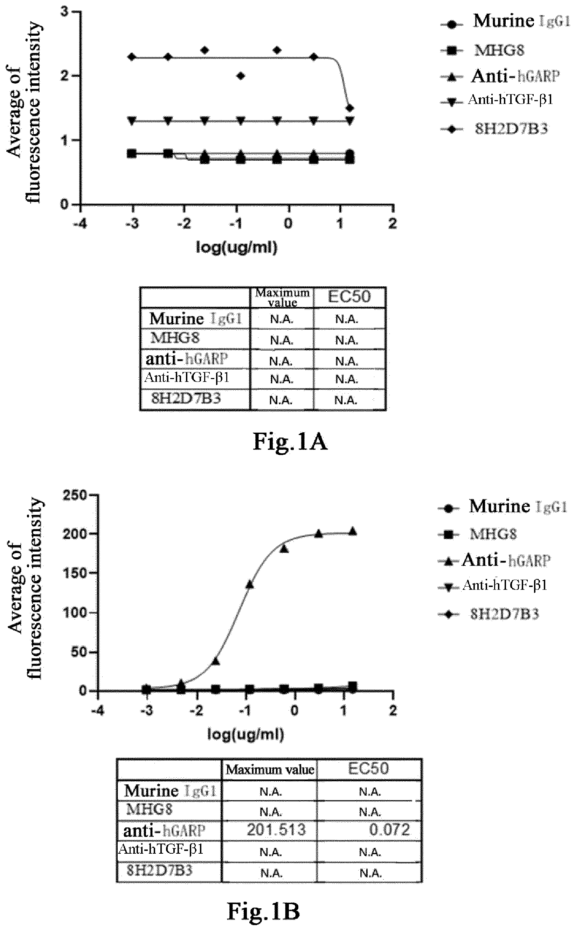

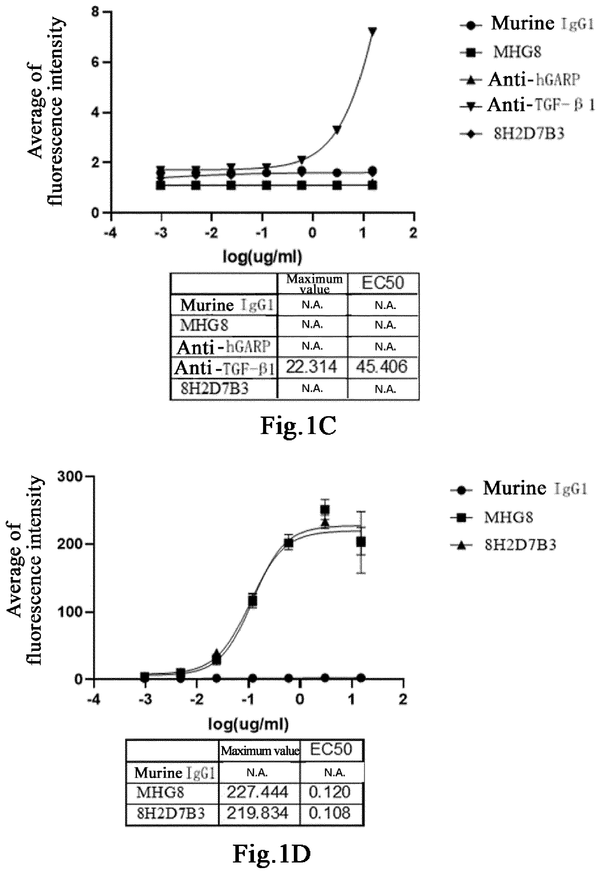

The cells were counted and centrifuged at 300 g for 5 min, the supernatant was discarded, the cell pellet was resuspended and washed with 1 × PBS, then centrifuged at 300 g for 5 min, the supernatant was discarded, and then the cell pellet was resuspended with FACS buffer containing 2% FBS to adjust to a density of 1e6 cells/mL. The cells were plated at a density of 1e5 cells/well for a constant volume of 100 µL/well at 4°C for 0.5 h, and centrifuged at 300 g for 5 min, and the supernatant was discarded. The antibody to be tested was prepared in FACS buffer containing 2% FBS with a starting concentration of 15 µg/mL, followed by serial dilution with a dilution factor of 1:5 in a total of 7 gradients, and then was added for a constant volume of 100 µL/well, with 0 µg/mL as a control, the dilution was pipetted and mixed thoroughly at 4°C for 1 h, and centrifuged at 300 g for 5 min, and the supernatant was discarded. 1 × PBS was added for a constant volume of 200 µL/well to resuspend and mix the cell pellet thoroughly, followed by centrifugation at 300 g for 5 min, the supernatant was discarded, and the steps were repeated twice. Secondary antibody Alexa 488 (donkey anti-mouse IgG (H+L), lot: 2018296, Thermo Fisher) was added, prepared in FACS buffer containing 2% FBS at a ratio of 1:1000 for a constant volume of 100 µL/well at 4°C, and the mixture was protected from light for 1 h. 1 × PBS was added for a constant volume of 200 µL/well, the cells were resuspended and mixed thoroughly, and centrifuged at 300 g for 5 min, the supernatant was discarded, and the steps were repeated twice. The cell pellet was resuspended in FACS buffer containing 2% FBS to read on a FACS machine. The software was used to map and calculate EC50. Wherein, MHG8 was used as a positive control, and mouse IgG1 (mIgG1) was used as an isotype control. The results were shown in Figs. 1A-1D: Fig. 1A shows the binding of the antibody to be tested to 293F cells, Fig. 1B shows the binding of the antibody to be tested to 293F-hGARP cells, Fig. 1C shows the binding of the antibody to be tested to 293F-hTGF-β1 cells, and Fig. 1D shows the binding of the antibody to be tested to 293F-hGARP/hTGF-β1 complex, indicating that the murine antibody 8H2D7B3 binds only to the 293F-hGARP/hTGF-β1 complex.

### 3.2 Flow cytometry assay of murine anti-human GARP monoclonal antibody for the blocking of the binding function of MHG8 (anti-human GARP antibody) to 293F-hGARP/hTGF-β1 complex

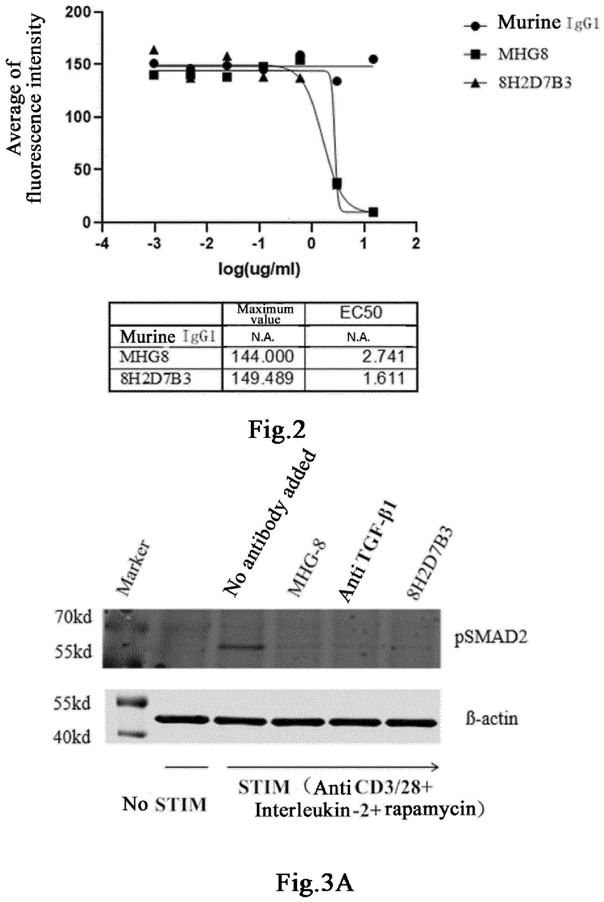

The cells were counted and centrifuged at 300 g for 5 min, the supernatant was discarded, the cell pellet was resuspended and washed with 1 × PBS, then centrifuged at 300 g for 5 min, the supernatant was discarded, and then the cell pellet was resuspended with FACS buffer containing 2% FBS to adjust to a density of 1e6 cells per mL. The cells were plated at a density of 1e5 cells per well for a constant volume of 100 µL/well at 4°C for 0.5 h, and centrifuged at 300 g for 5 min, and the supernatant was discarded. The competitor antibody with alexa488 (lot: 1905151702, 1.39 mg/mL) was added to a final concentration of 30 µg/mL for a constant volume of 50 µL/well; the antibody to be tested was prepared in FACS buffer containing 2% FBS with a starting concentration of 30 µg/mL, followed by serial dilution with a dilution factor of 1:5 in a total of 7 gradients, and then was added for a constant volume of 100 µL/well, with 0 µg/mL as a control, the mixture was pipetted and mixed thoroughly at 4°C for 1 h, and centrifuged at 300 g for 5 min, and the supernatant was discarded. 1 × PBS was added for a constant volume of 200 µL/well to resuspend and mix the cell pellet thoroughly, followed by centrifugation at 300 g for 5 min, the supernatant was discarded, and the steps were repeated twice. Secondary antibody Alexa 488 (donkey anti-mouse IgG (h+L), lot: 2018296, Thermo Fisher) was prepared in FACS buffer containing 2% FBS at a ratio of 1:1000, then added for a constant volume of 100 µL/well at 4°C, and the mixture was protected from light for 1 h. 1 × PBS was added for a constant volume of 200 µL/well to resuspend and mix thoroughly the cells, followed by centrifugation at 300 g for 5 min, the supernatant was discarded, and the steps were repeated twice. The cell pellet was resuspended in FACS buffer containing 2% FBS and loaded on a FACS machine to read experiment data, which was mapped with a software to calculate EC50. Wherein, MHG8 was used as a positive control, and mouse IgG1 (mIgG1) was used as an isotype control. Results were as shown in Fig. 2, murine antibody 8H2D7B3 could block the binding of MHG8 to the 293F-hGARP/hTGF-β1 complex.

### 3.3 Western blot and Alpha Elisa assay of the inhibition of SMAD2 phosphorylation in Treg cells by purified murine anti-human GARP monoclonal antibodies

Fresh PBMC cells were purchased from ALLCELLS, firstly PBMC cells were centrifuged at 400 g for 10 min, then resuspended in 1× PBS medium (Thermo Fisher, 10010049) containing 1% FBS (Thermo Fisher, 10099-141) and 50 mmol EDTA (Thermo Fisher, 15400054). PBMC was subjected to Treg cell extraction according to a Treg Extraction Kit (Miltenyi, Cat. No.: 130-091-301) protocol. Treg cell antibody treatment: Treg were expanded in vitro for 14 days, cells were collected and counted, and 1.5×10⁶ Treg cells were depleted of magnetic beads and used as a negative control. The cells were plated in a 24-well plate at 1×10⁶ /mL per well for a constant volume of 1 mL/well. The antibody to be tested was added to the 24-well plate at a concentration of 25 µg/mL. The cells were cultured 36 h in a carbon dioxide incubator at 37°C.

Alpha Elisa assay: the cells were collected by centrifugation, and 50 uL of Lysis buffer was added for lysis 30 min. The lysate was centrifuged at 1300 rpm for 5 min, and 10 uL supernatant was fetched for Alpha Elisa assay (PerkinElmer, ALSU-PSM2-A500).

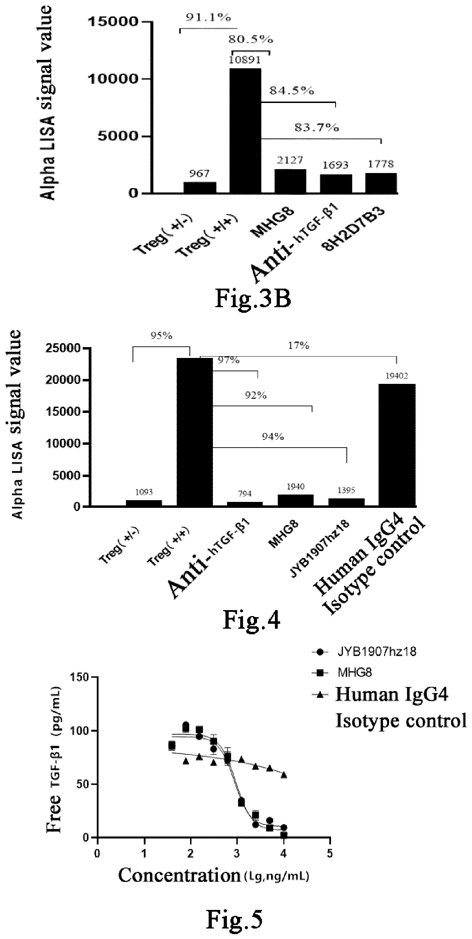

Western blot assay: the cells were collected by centrifugation, and 50 uL of Lysis buffer was added for lysis 30 min. The lysate was centrifuged at 1300 rpm for 5 min, and 10 uL supernatant was fetched for Western blot assay. The results were shown in Figs. 3A-3B: Fig. 3A shows the Western blot assay results of the inhibition of SMAD2 phosphorylation in Treg cells by the antibody to be tested, and Fig. 3B shows the Alpha Elisa assay signal value of the inhibition of SMAD2 phosphorylation in Treg cells by the antibody to be tested, indicating that the murine antibody was similar to the positive controls anti-TGF-β1 and MHG8, both of which could inhibit the SMAD2 phosphorylation protein in Treg cells.

By functional identification such as binding and blocking, 8H2D7B3 was selected as murine candidate antibody and humanized.

The sequence of the variable region of the murine anti-human GARP monoclonal antibody 8H2D7B3 was determined as follows:
>8H2D7B3 VH 
QVQLQQSGAELVRPGSSVKLSCKASGYTLSNYWMYWVKQRPIQGLEWIGSIAPSDSE
THYNQKFKDRATVTVDKSSRIVYMQLSSLTSEDSAVYFCARGGFGYGSSHWYFDVWGTGT
TVTVSS
>8H2D7B3 VL

DIQMTQSSAYLSVSLGGRVTITCKASDHINKWLAWYQQKPGNAPRLLISGATSLETGIP
SRFSGSGSGKDYTLSITGLQTEDVATYYCQQYWTTPYTFGGGTKLEIK

The CDR regions of the murine anti-human GARP monoclonal antibody 8H2D7B3 heavy and light chains were shown in Table 3.

**Table 3 CDR regions of murine anti-human GARP monoclonal antibody 8H2D7B3 heavy and light chains**

| Heavy chain | | Light chain | |
|---|---|---|---|
| HCDR1 | GYTLSNY | LCDR1 | KASDHINKWLA |
| HCDR2 | APSDSE | LCDR2 | GATSLET |
| HCDR3 | GGFGYGSSHWYFDV | LCDR3 | QQYWTTPYT |

### Example 4 Humanization of murine anti-human GARP monoclonal antibody

4.1 The human Germline antibody (data source: IMGT) that is most homologous to the murine anti-human GARP monoclonal antibody 8H2D7B3 was selected by sequence alignment as a framework for humanization design (the light chain was framed with IGKV3D-15*01 or IGKV1-5*03, IGKJ2*02 or IGKJ4*02, and the heavy chain was framed with IGHV3-7*03 or IGHV1-46*03, IGHJ3*01), the CDR regions of the antibody were defined by Chothia numbering [Chothia & Lesk, 1987] of the variable regions of the antibody light and heavy chains: CDRL1(L24-L34), CDRL2(L50-L56), CDRL3(L89-L97), CDRH1(H26-H32), CDRH2(H52-H56), and CDRH3(H95-H97), amino acids in the variable region of the antibody light and heavy chains were subjected to humanization mutations according to sequence alignment and structural information of the variable regions.

### 4.2 Germline antibody sequence information

IGKV3D-15*01: EIVMTQSPATLSVSPGERATLSCRASQSVSSNLAWYQQKPGQAPRLLIYGASTRATGIPA RFSGSGSGTEFTLTISSLQSEDFAVYYCQQYNNWP
IGKV 1-5 *03 : 
DIQMTQSPSTLSASVGDRVTITCRASQSISSWLAWYQQKPGKAPKLLIYKASSLESGVP
SRFSGSGSGTEFTLTISSLQPDDFATYYCQQYNSYS
IGHV3-7*03: 
EVQLVESGGGLVQPGGSLRLSCAASGFTFSSYWMSWVRQAPGKGLEWVANIKQDGSE
KYYVDSVKGRFTISRDNAKNSLYLQMNSLRAEDTAVYYCAR
IGHV1-46*03: 
QVQLVQSGAEVKKPGASVKVSCKASGYTFTSYYMHWVRQAPGQGLEWMGIINPSGG
STSYAQKFQGRVTMTRDTSTSTVYMELSSLRSEDTAVYYCAR
IGKJ2*02:
   CTF GQGTKLEIK
IGKJ4*02:
   LTFGGGTKVEIK
IGHJ3*01:
   D AFD VWGQGTMVT VS S

4.3 The expression vector is designed and the gene is synthesized. Then the recombinant antibodies are expressed in mammalian cells and purified. The humanized antibody and chimeric antibody were compared in terms of activity and physicochemical properties, and 1-2 rounds of humanization optimization were performed, wherein the light and heavy chains are optimally designed by using the humanized sequence grafted by the Germinne antibody as a framework CDR. The resulting chimeric antibody was named as JYB1907hz0 and the humanized antibody was named as JYB1907hz18.

### 4.4 Expression and purification of humanized antibody

After optimizing the designed sequence by antibody engineering, the full-length JYB1907hz18 light and heavy chain protein sequence was codon-optimized, respectively, the codon-optimized DNA fragment (Genscript) was synthesized, and the synthesized gene fragment was cloned into expression vector pcDNA3.4 (Life Technologies). After expression plasmid amplification and plasmid extraction, ExpiCHO cells (ThermoFisher Scientific, A29133) were co-transfected with two plasmids and transient antibody expression was performed according to the supplier's ExpiCHO expression system method. The approximate procedure was as follows: ExpiCHO cells were cultured to a density of 6×10⁶/mL in a total culture volume of 25 ml culture medium at 36.5°C and 8% carbon dioxide concentration,. 10 µg of antibody light and heavy chain expression plasmids were respectively transferred to the cells using ExpiFectamine transfection reagent; one day after the transfection, 150 µL of ExpiCHO enhancer and 4 mL of ExpiCHO adjuvant was each added to the cultured cells, and the culture was continued for 9 days. The supernatant was obtained by centrifugation at 3500 rpm and 4°C. AmMagTM Protein A magnetic beads (Genscript, L00695) and antibody expression supernatant were mixed, the mixture was incubated at room temperature for 2 h, and washed twice with PBS, the supernatant was discarded; an appropriate amount of elution buffer Protein G or A SefinoseTMElution buffer (Sangon, C600481) was added, the mixture was mixed thoroughly, and then placed on a test tube rack for static incubation for 5 min; the magnetic beads were resuspended for 2-3 times during the incubation, and the elution was repeated for 2 times. After elution, the elute was neutralized with an appropriate amount of neutralizing solution 1M Tris-HCl, pH7.5 (Sangon, B548124) immediately for later use .

### 4.5 Affinity assay of purified humanized antibody

4.5.1 The affinity of JYB1907hz18 and hGARP/hTGF-β1 complex (Kactus Biosystems, Cat. No.: GAR-HM4TG) was measured by Octet RED96e (Fortebio), both of the 1907Hz18 and hGARP/hTGF-β1 complex were diluted with 1×PBST (1×PBS: Sangon Biotech, B548117-0500; 0.02% Tween 20: Sigma, P1379), wherein the hGARP/hTGF-β1 complex was diluted in a 3-fold gradient from a starting concentration of 100 nM, and JYB1907hz18 was used at a concentration of 33.3 nM.

4.5.2 Loading sample for detection (Octet Data Acquisition 11.1.0.11): firstly, the sample was added to a 96-well plate (Greiner bio-one, 655209) in a system of 200 µL/well. Software parameters were then set, the plate temperature was set to 30°C, and the frequency at which the standard kinetic signal was collected was 5.0 HZ. Next, the ARC sensor (Fortébio, Cat. No.: 18-0015) was pre-wetted with 1×PBST for 10 min, then the sample was loaded on the machine for detection. Each cycle contains the following steps: 1) immersing the sample in buffer for 60 s; 2) detecting whether the antigen (hGARP/hTGF-β1 complex) non-specifically binding to the sensor; 3) regenerating in a 1.0 mM glycine solution at pH 1.7; 4) immersing in buffer for 60 s; 5) immobilizing the antibody (JYB1907hz18) on the sensor for 25 s; 6) immersing the sensor in buffer solution for 180 s; 7) binding the antigen (hGARP/hTGF-β1 complex) to the antibody (JYB1907hz18) for 180 s; 8) dissociating the antigen (hGARP/hTGF-β1 complex) with antibody (JYB1907hz18) for 10 min; 9) regenerating the sensor.

### 4.5.3 Data analysis

The equilibrium dissociation constant (KD) of the antibody was calculated by measuring the association rate (Ka) and dissociation rate (Kd) of the antigen (hGARP/hTGF-β1 complex)-antibody (JYB1907hz18) in a 1:1 binding mode using Data Analysis 12.0 software from Fortebio. Results were as shown in Table 4, JYB1907hz18 bound to hGARP/hTGF-β1 with greater affinity than the positive control antibody MHG8.

**Table 4 Affinity assay results of JYB1907hz18 and hGARP/hTGF-β1**

| Candidate antibodies | K_{D}(M) | ka (1/Ms) | kd (1/s) |
|---|---|---|---|
| JYB1907hz18 | <1.0E-12 | 1.95E+05 | <1.0E-07 |
| MHG8 | 2.05E-10 | 1.39E+05 | 2.85E-05 |

### Example 5 Inhibition of SMAD2 phosphorylation in Treg cells by JYB1907hz18

Fresh PBMC cells were purchased from ALLCELLS, firstly PBMC cells were centrifuged at 400 g for 10 min, then resuspended in 1× PBS medium (Thermo Fisher, 10010049) containing 1% FBS (Thermo Fisher, 10099-141) and 50 mmol EDTA (Thermo Fisher, 15400054). PBMC was subjected to Treg cell extraction according to a Treg Extraction Kit (Miltenyi, Cat. No.: 130-091-301) protocol. Treg cell antibody treatment: Treg were expanded in vitro for 14 days, cells were collected and counted, and 1.5×10⁶ Treg cells were depleted of magnetic beads and used as a negative control. The cells were plated in a 24-well plate at 1×10⁶ /mL per well for a constant volume of 1 mL per well. The antibody to be tested was added to the 24-well plate at a concentration of 25 µg/mL. The cells were cultured 36 h in a carbon dioxide incubator at 37°C. Wherein, anti-TGF-β1 and MHG8 were used as positive controls, and human IgG4 was used as an isotype control.

Alpha Elisa assay: the cells were collected by centrifugation, and 50 uL of Lysis buffer was added for lysis 30 min. The lysate was centrifuged at 1300 rpm for 5 min, and 10 uL supernatant was fetched for Alpha Elisa assay (PerkinElmer, ALSU-PSM2-A500).

JYB 1907hz 18 inhibited SMAD2 phosphorylation in Treg cells as assessed by the method described above, with the results shown in Fig. 4.

### Example 6 Assay study of inhibition of TGFβ1 release by JYB1907hz18

293T-hGARP/hTGF-β1 cells were constructed by Shanghai ChemPartner Co., Ltd., and LN229 cells were purchased from Nanjing Cobai (Cat. No.: CBP60302). When the two kinds of cells were grown to a density of 70%, they were digested with trypsin (Thermo, 25200056), respectively, fresh DMEM (Thermo Fisher, 11965092) containing 10% FBS (Thermo Fisher, 10099-141) and 1 × Pen Strep (Thermo Fisher, 15140-122) was added to resuspend each cell, and 3 × 10⁶ cells per well (50 µL/well) were sequentially added to a 96-well plate (Corning, 3599). 50 µL of JYB1907hz18 diluted in a gradient manner (2-fold serial dilution from a final concentration of 10 µg/mL in a total of 10 gradients, including an antibody concentration of 0 for the final well) was added into each well, and the mixture was incubated in a 37°C, 5% CO₂ incubator for 5-6 h. The culture was centrifuged, and the supernatant was fetched and subjected to TGF-β1 assay with a TGF-β1 ELISA kit (Biolegend, Cat. No.: 580709). Wherein, MHG8 was used as a positive control, and human IgG4 was used an isotype control.

JYB1907hz18 inhibited the release of TGF-β1 from 293T cells as assessed by the method described above, with the results shown in Fig. 5.

### Example 7 JYB1907hz18 preventing Treg from inhibiting the release of IL-2 and IFN γ from human peripheral blood mononuclear cells (PBMC)

Fresh PBMC cells were purchased from TPCS, firstly PBMC cells were centrifuged at 400 g for 10 min, then resuspended in 1×PBS medium (ThermoFisher, 10010049) containing 1% FBS (ThermoFisher, 10099-141) and 50 mmol EDTA (ThermoFisher, 15400054). A few PBMC were pipetted for subsequent experiments, and the remaining PBMCs were subjected to Treg cell extraction according to a Treg Extraction Kit (Miltenyi, Cat. No.: 130-091-301) protocol. The extracted Treg cells and PBMC cells were counted and adjusted to a concentration of 5e5/mL, the PBMC group was supplemented with 50 µL of PBMC, 48 µL of TexMACS^{™} Medium medium (Miltenyi Biotec, 170-076-309) containing 1% HBS (TPCS, A515) and 1×Pen Strep (ThermoFisher, 15140-122), and 2 µL of CD3/CD28 antibody magnetic beads (Miltenyi Biotec, 130-095-345). The PBMC+Treg cell group was supplemented with 50 µL of PBMC, 46 µL of TexMACS^{™} Medium medium (Miltenyi Biotec, 170-076-309) containing 1% HBS (TPCS, A515) and 1×Pen Strep (ThermoFisher, 15140-122), and 4 µL of CD3/CD28 antibody magnetic beads (Miltenyi Biotec, 130-095-345). The antibody to be tested, JYB1907hz18, was added for a constant volume of 50 µL/well at a final concentration of 25 µg/ml. The mixture was incubated in a 37°C, 5% CO₂ incubator for 20-24 h. The culture was centrifuged, and the supernatant was fetched and assayed for IL-2 (R&D, VAL110) and IFN-γ (R&D, DIF50C) using IL-2 ELISA and IFN-y ELISA kits. Wherein, MHG8 was used a positive control.

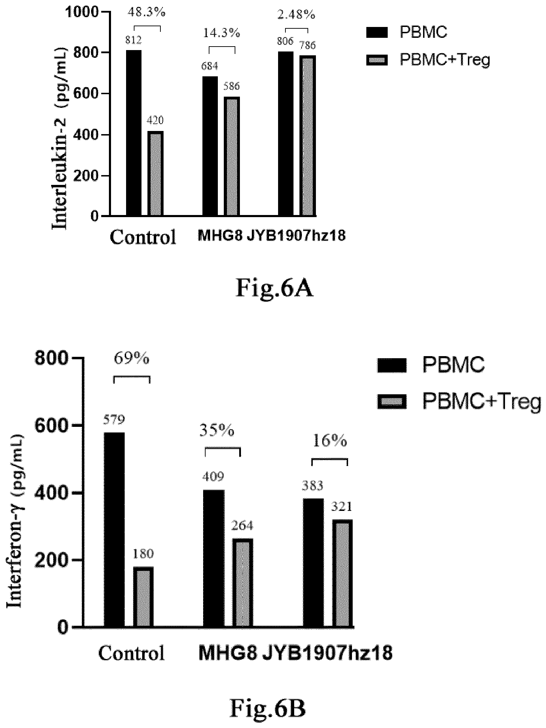

JYB1907hz18 inhibited the release of interleukin -2 (IL-2) and interferon-γ (IFN-γ) from peripheral blood mononuclear cells (PBMCs) as assessed by the methods described above, with the results shown in Figs. 6A-6B: Fig. 6A shows that the antibody to be tested prevents Treg from inhibiting the release of interleukin-2 (IL-2) from PBMC; Fig. 6B shows that the antibody to be tested prevents Treg from inhibiting the release of interferon-γ (IFN-γ) from PBMC.

### Example 8 Pharmacokinetic (PK) studies in humanized FcRn mouse models

Humanized FcRn mice were used as test animals, after single subcutaneous administration, the pharmacokinetic indexes of two drugs to be tested, MHG8 and JYB 1907hz 18, were studied, respectively. All animal experimental schemes were reviewed and approved by IACUC. Male hFcRn mice aged between 6 and 8 weeks were purchased from Biocytogen Pharmaceuticals (Beijing) Co., Ltd., with body weight of 23-26 g, raised in SPF-grade animal room, fed with standard pellet feed, with free access to food and water at room temperature of 18-24°C and relative humidity of 40%-50%, exposed to daily alternating light-dark cycles (i.e., 12 h shifts). A total of 12 experimental animals were randomly divided into three groups, with four animals in each group. The animals were subcutaneously administered with a single dose of 10 mg/kg at a dose volume of 10 mL/kg. Blood was sampled before the drug administration, and 2 h, 6 h, 24 h (day 1), day 2, day 3, day 4, day 7, day 10, day 14, day 21, day 28, day 35, and day 42 after the drug administration. Whole blood was collected into EP tubes at 60 µL/tube by puncturing cheek, allowed to stand at room temperature for 30 min, and then centrifuged (2000 g, 4°C, 5 min) to separate serum. Each sample was divided into 2 portions (a detection tube and a backup tube) at 10 µL/tube, and stored at -80°C.

Pharmacokinetics of three drugs at different time points were analyzed by indirect ELISA. The plate was coated with the antigen, murine anti-human IgG4 Fc (Abcam, lot: GR3248093-2, ab99820) at 2 µg/mL, 100 µL/well, overnight at 4°C, then washed, and blocked with a blocking buffer at 200 µL/well overnight at 4°C. Serum sample was added at 37°C for 1 h, for a constant volume of 50 µl/well. The detection antibody, hGARP/hTGF β1-biotin (0.78 mg/ml, lot: 030201) + Streptavidin-peroxidase (Sigma, lot: SLCB5784), was added for 0.5 h at 37°C for a constant volume of 100 µ/well. TMB developing solution (KPL, Cat. No.: 52-00-03) was added for color development, OD450 was read on a microplate reader (Molecular Devices, SpectraMax M3). Drug concentration was obtained according to a standard curve, and PK parameters were obtained by PK Solver non-compartmental data processing.

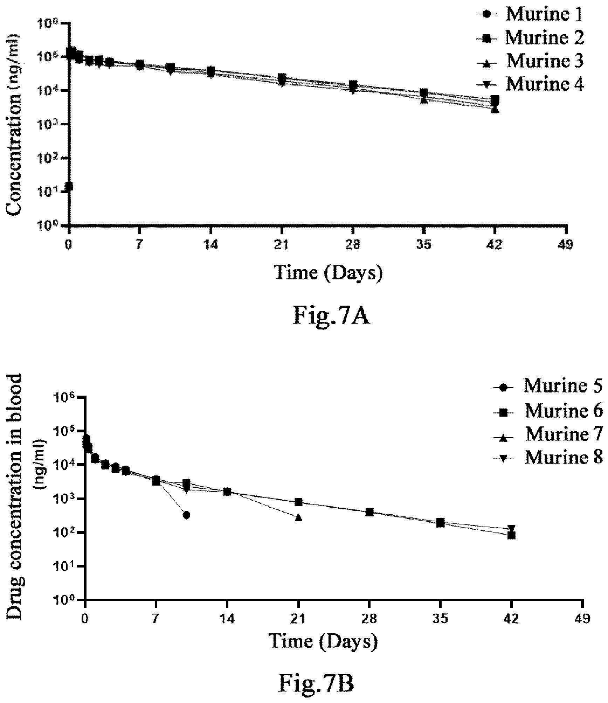

The half-life (t1/2) after administration of MHG8 = 4.8 days, and the maximum concentration (Cmax) was 50036 ng/ml (as shown in Fig. 7B); the half-life (t1/2) after administration of JYB1907hz18 = 8.82 days, with the maximum concentration (Cmax) of 139437ng/ml (as shown in Fig. 7A).

### Example 9 Study on physicochemical properties of JYB1907hz18

### 9.1 SEC-HPLC Purity analysis

9.1.1 The sample (JYB1907hz18) was diluted to 1 mg/mL, mixed thoroughly, and centrifuged at 12000 rpm for 5 min, the supernatant was transferred to a sample bottle, and placed in a HPLC sample tray. The chromatographic conditions were set as shown in Table 5.

**Table 5 Chromatographic conditions**

| Chromatographic conditions | Parameters |
|---|---|
| Chromatographic column | TSK |
| | G3000SWxl |
| Detection wavelength | 280 nm |
| Column temperature | 25°C |
| Sample room temperature | 5°C |
| Flow rate | 0.5 ml/min |

9.1.2 The chromatographic column was equilibrated with mobile phase (200 mM phosphate buffer, pH 6.8), the sample was injected for analysis. The chromatographic software was used for data analysis. The peak area percentage of each peak was calculated by peak area normalization method.

### 9.2 HIC-HPLC Analysis

9.2.1 The sample (JYB1907hz18) was diluted to 1 mg/ml and the supernatant was centrifuged for assay. The chromatographic conditions were set as shown in Table 6.

**Table 6 Chromatographic conditions**

| Chromatographic conditions | Parameters |
|---|---|
| Chromatographic column | MAbPac^{™}HIC-1 |
| | 0 |
| Detection wavelength | 214 nm |
| Column temperature | 30°C |
| Sample room temperature | 5°C |
| Flow rate | 0.8 ml/min |

9.2.2 Gradient elution was performed with mobile phase A (50 mM phosphate buffer/1M ammonium sulfate, pH 7.0) and mobile phase B (50 mM phosphate buffer, pH 7.0), and the main peak retention time was recorded.

### 9.3 Analysis of melting temperature (Tm) value

The sample (JYB1907hz18) was diluted with sample buffer to 1 mg/mL, then according to the instructions of Protein Thermal Shift^{™} Starter Kit, 13 µL of sample (JYB1907hz18) solution was added into a PCR tube, 5 µL of Protein Thermal shift TM Buffer was added, 2 µL of 10× staining solution was added, resulting a reaction volume of 20 µL, the mixture was mixed thoroughly, and centrifuged at 12000 rpm for 5 min to remove air bubbles. The test sample (JYB1907hz18) was placed in the PCR instrument for sample analysis, and the Tm value of sample (JYB1907hz18) was recorded.

### 9.4 iCIEF Analysis

The sample (JYB1907hz18) solution was added into the following thoroughly mixed system: 70 µL of 1% methylcellulose (MC), 80 µL of 5M urea, 8 µL of amphoteric electrolyte Pharmalyte pH 3-10, 2 µL of pI marker 5.5, 2 µL of pI marker 9.5. The system was supplemented with appropriate volume of ultrapure water to 200 µl, and the mixture was mixed thoroughly, then centrifuged, and the supernatant was analysized. After the analysis, the result file was imported into ChromPerfect software for spectrum integration processing and calculation of the isoelectric point of each peak as well as the percentage of each peak.

The results for physicochemical properties analysis of JYB1907hz18 were shown in Table 7.

**Table 7 Results for physicochemical properties analysis of JYB1907hz18**

| Protein Name | Physicochemical Properties | | | | | | |
|---|---|---|---|---|---|---|---|
| | SEC% | Tm °C | HIC (min) | Measured pI | Acid Peaks (%) | Main Peak (%) | Basic Peaks (%) |
| | | Fab | | | | | |
| JYB1907hz18 | 99.5 | 80.1 | 15.06 | 7.9 | 11.8 | 74.0 | 14.2 |

### Example 10 Study on JYB1907hz18 antibody in mouse GvHD model

40 female NOG mice aged between 7 and 8 weeks were purchased from Beijing Vital River Laboratory Animal Technology Co., Ltd.; Cryopreserved PBMC was purchased from Miaotong (Shanghai) Biotechnology Co., Ltd.; Treg cells were extracted from fresh PBMC and expanded in vitro as described in Example 5. After NOG mice were acclimated, mice were treated with 25 mg/kg of busulfan intraperitoneally 3 days in advance. Ten mice were randomly selected, and cryopreserved PBMCs from the same batch were recovered. Each mouse was intravenously injected with 3×10⁶ PBMC as the first group. The recovered PBMCs were mixed with Treg cells, and the remaining 30 mice were injected intravenously with 3×10⁶ PBMCs and 3×10⁶ Treg cells via the tail vein. On the day after injection, the mice were randomly divided into groups 2, 3, and 4 according to body weight. After grouping, the mice in the first group was intraperitoneally injected with isotype Ctrl, the mice in the second group was intraperitoneally injected with isotype Ctrl, the mice in the third group was intraperitoneally injected with MHG8 (positive control) at a dose of 20 mg/kg, and the mice in the fourth group was intraperitoneally injected with JYB1907hz18 at a dose of 20 mg/kg, with administration frequency of twice a week, for 12 times in total. Mice were weighed twice weekly and subjected to GvHD scoring, and survival was counted. The GvHD scoring criteria were shown in Table 8. Mice were euthanized when they lost more than 25% of their body weight.

**Table 8 GvHD scoring criteria**

| | | | |
|---|---|---|---|
| Loss of weight | -5% | -15% | -25% |
| | 1 score | 2 scores | 3 scores |
| Jaundice or anemia | 1 score | | |
| Hollow back | 1 score | | |
| Range of motion | Decreased range of motion | Inactive | |
| | 1 score | 2 scores | |
| Hair loss/blowing | 1 score | | |
| Death | | 8 scores | |

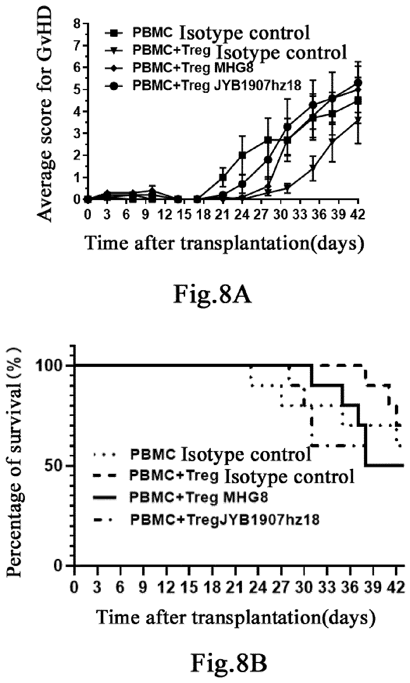

By the end of the experiment on day 42, 4 mice were found dead in the first group, 3 mice were found dead in the second group, and 5 mice survived in the third and fourth groups. In the second group, since the addition of Treg cells, the expansion of PBMC in vivo was inhibited, the occurrence time of GvHD was delayed, while the administration of MHG8 and JYB1907hz18 in the third and fourth groups inhibited the effect of Treg, and the scores and mortality rates of GvHD were higher than those in the first group, with the results were shown in Figs. 8A-8B: Fig. 8A shows the GvHD score of the antibody to be tested in mice; Fig. 8B shows the survival rate of the antibody to be tested in mice.

### Example 11 Pharmacodynamic testing of the JYB1907hz18 antibody in the human melanoma A375 mouse tumor model

48 female NOG-DKO mice aged between 7 and 8 weeks were purchased from Beijing Vital River Laboratory Animal Technology Co., Ltd.; cryopreserved PBMC was purchased from Miaotong (Shanghai) Biotechnology Co., Ltd.; A375 human melanoma cells were purchased from ATCC. The PBMC were recovered and PBMC was injected into each mouse via the tail vein in an amount of 6E6 to establish a humanized mouse model. One week later, the expanded cultured A375 cells were collected and mixed with matrigel, and then inoculated subcutaneously into NOG-DKO mice in an amount of 2E6. When the tumors grew to a volume of 100 mm³, mice were grouped randomly, with 8 mice in each group, and administered on the same day. Group G1 was administered with an isotype control at a dose of 20 mg/kg; group G2 was administered with MHG8 (positive control) at a dose of 20 mg/kg; group G3 was administered with JYB1907hz18 at a dose of 20 mg/kg; group G4 was administered with Tencentriq at a dose of 10 mg/kg; group G5 was administered with MHG8 (at a dose of 20 mg/kg) and Tencentriq (at a dose of 10 mg/kg); group G6 was administered with JYB1907hz18 (at a dose of 20 mg/kg) and Tencentriq (at a dose of 10 mg/kg). All mice were administrated intraperitoneally twice a week for a total of 8 doses.

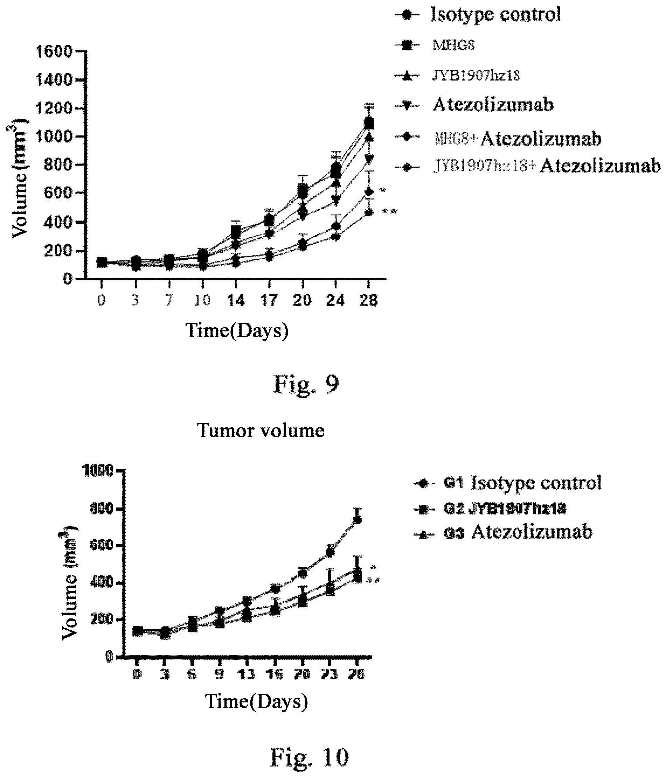

On Day 28 after grouping, the mean tumor volume in G1 group was 1110 ± 104 mm³, the mean tumor volume in G2 group was 1088 ± 116 mm³, the mean tumor volume in G3 group was 1000 ± 233 mm³, the mean tumor volume in G4 group was 834 ± 155 mm³, the mean tumor weight in G5 group was 611 ± 146 mm³, the mean tumor volume in G6 group was 471 ± 87 mm³, the tumor inhibition rate in G5 group was 50%, and the tumor inhibition rate in G6 group was 64%. Both G5 and G6 groups were significantly different from G1 group (G5 vs G1, G6 vs G1), G5 vs G1, P <0.05; G6 vs G1, P <0.001. There was no statistical difference in other groups. The combination of MHG8 with Tecentriq (anti-PD-L1 antibody) and JYB1907hz18 with Tecentriq (anti-PD-L1 antibody) were demonstrated to inhibit tumor growth, and the results were shown in Fig. 9.

### Example 12 Pharmacodynamic study of JYB1907hz18 antibody in a human breast cancer JIMT-1 mouse tumor model

24 female NOG-DKO mice aged between 7 and 8 weeks were purchased from Beijing Vital River Laboratory Animal Technology Co., Ltd.; cryopreserved PBMC was purchased from Miaotong (Shanghai) Biotechnology Co., Ltd.; JIMT-1 human breast cancer cells were purchased from ATCC. The PBMC were recovered and PBMC was injected into each mouse via the tail vein in an amount of 6E6 to establish a humanized mouse model. One week later, the expanded cultured JIMT-1 cells were collected and mixed with matrigel, and then the mixture was inoculated subcutaneously into NOG-DKO mice via the back in an amount of 3E6. When the tumor volume was about 100 mm³, mice were grouped randomly, with 8 animals in each group, which were administered on the same day. Group G1 was administered with an isotype control at a dose of 20 mg/kg; group G2 was administered with JYB1907hz18 at a dose of 20 mg/kg; group G3 was administered with Tecentriq at a dose of 10 mg/kg. All mice were administrated intraperitoneally twice a week for a total of 8 doses.

On Day 28 after grouping, the mean tumor volume in G1 group was 740 ± 57 mm³, the mean tumor volume in G2 group was 426 ± 45 mm³, the mean tumor volume in G3 group was 469 ± 72 mm³, the tumor inhibition rate in G2 group was 52%, and the tumor inhibition rate in G3 group was 45%. Both of G2 and G3 groups were significantly different from G1 group (G2 vs G1, G3 vs G1), G2 vs G1, P <0.001; G3 vs G1, P<0.01. Both JYB1907hz18 alone and Tecentriq (anti-PD-L1 antibody) alone were demonstrated to inhibit tumor growth, with JYB1907hz18 had better tumor growth inhibition effect than Tecentriq (anti-PD-L1 antibody), and the results were shown in Fig. 10.

### Example 13 Pharmacodynamic study of the JYB1907hz18 antibody in a human lung squamous cell carcinoma EBC-1 mouse tumor model

24 female NOG-DKO mice aged between 7 and 8 weeks were purchased from Beijing Vital River Laboratory Animal Technology Co., Ltd.; cryopreserved PBMC was purchased from Miaotong (Shanghai) Biotechnology Co., Ltd.; EBC-1 human breast cancer cells were purchased from ATCC. The PBMC were recovered and PBMC was injected into each mouse via the tail vein in an amount of 6E6 to establish a humanized mouse model. One week later, the expanded cultured EBC-1 cells were collected and mixed with matrigel, and then the mixture was inoculated subcutaneously into NOG-DKO mice via the back in an amount of 3E6. When the tumor volume was about 100 mm³, mice were grouped randomly, with 8 animals in each group, which were administered on the same day. Group G1 was administered with an isotype control at a dose of 20 mg/kg; group G2 was administered with JYB1907hz18 at a dose of 20 mg/kg; group G3 was administered with Tecentriq at a dose of 10 mg/kg. All mice were administrated intraperitoneally twice a week for a total of 8 doses.

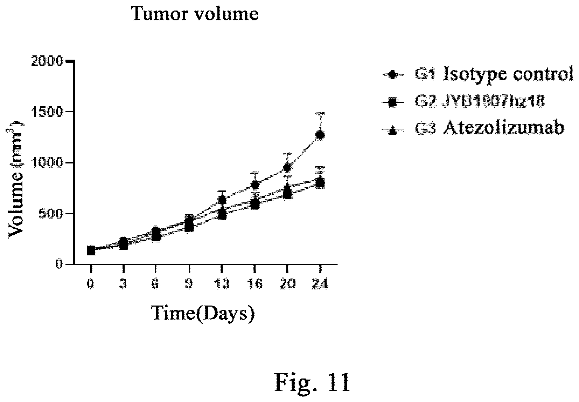

On day 24 after grouping, the mean tumor volume in G1 group was 1273 ± 212 mm³, the mean tumor volume in G2 group was 797 ± 113 mm³, the mean tumor volume in G3 group was 842 ± 115 mm³, the tumor inhibition rate in G2 group was 42%, and the tumor inhibition rate of G3 group was 38%. From the tumor growth trend graph, both JYB1907hz alone and Tecentriq (anti-PD-L1 antibody) alone tended to inhibit tumor growth on this cancer species, and the results were shown in Fig. 11.

## Claims

1. An isolated antigen-binding protein having one or more of the following properties:
a) binding to the human GARP/human TGF-β1 complex in an Octet assay with a *K_{D}* value of about 1.0E-12 or less;
b) not binding to cells expressing only human GARP or cells expressing only human TGF-β1;
c) inhibiting the SMAD2 phosphorylation in Treg cells;
d) inhibiting the release of TGF-β1 from HEK293T cells expressing human GARP/human TGF-β1;
e) preventing Treg cells from inhibiting the release of IL-2 and IFN- γ from human peripheral blood mononuclear cells (PBMC); and
f) blocking the inhibitory effect of Treg cells on GvHD induced by the human PBMCs in a human PBMC transplanted mice GvHD model.

2. The isolated antigen-binding protein of claim 1, comprising HCDR3 comprising an amino acid sequence as set forth in SEQ ID NO: 4.

3. The isolated antigen-binding protein of any one of claims 1-2, comprising HCDR2 comprising an amino acid sequence as set forth in SEQ ID NO: 3.

4. The isolated antigen-binding protein of any one of claims 1-3, comprising HCDR1 comprising an amino acid sequence as set forth in SEQ ID NO: 2.

5. The isolated antigen-binding protein of any of claims 1-4, comprising a heavy chain variable region VH which comprises the HCDR1 comprising an amino acid sequence as set forth in SEQ ID NO: 2, the HCDR2 comprising an amino acid sequence as set forth in SEQ ID NO: 3, and the HCDR3 comprising an amino acid sequence as set forth in SEQ ID NO: 4.

6. The isolated antigen-binding protein of any one of claims 1-5, comprising H-FR1, wherein the C-terminus of the H-FR1 is directly or indirectly linked to the N-terminus of the HCDR1, and the H-FR1 comprises an amino acid sequence as set forth in SEQ ID NO: 46.

7. The isolated antigen-binding protein of claim 6, wherein the H-FR1 comprises an amino acid sequence as set forth in any one of SEQ ID NO: 5 and SEQ ID NO: 22.

8. The isolated antigen-binding protein of any one of claims 1-7, comprising H-FR2, wherein the H-FR2 is located between the HCDR1 and the HCDR2, and the H-FR2 comprises an amino acid sequence as set forth in SEQ ID NO: 47.

9. The isolated antigen-binding protein of claim 8, wherein the H-FR2 comprises an amino acid sequence as set forth in any one of SEQ ID NO: 6 and SEQ ID NO: 23.

10. The isolated antigen-binding protein of any one of claims 1-9, comprising H-FR3, wherein the H-FR3 is located between the HCDR2 and the HCDR3, and the H-FR3 comprises an amino acid sequence as set forth in SEQ ID NO: 48.

11. The isolated antigen-binding protein of claim 10, wherein the H-FR3 comprises an amino acid sequence as set forth in any one of SEQ ID NO: 7 and SEQ ID NO: 24.

12. The isolated antigen-binding protein of any one of claims 1-11, comprising H-FR4, wherein the N-terminus of the H-FR4 is directly or indirectly linked to the C-terminus of the HCDR3, and the H-FR4 comprises an amino acid sequence as set forth in SEQ ID NO: 49.

13. The isolated antigen-binding protein of claim 12, wherein the H-FR4 comprises an amino acid sequence as set forth in any one of SEQ ID NO: 8 and SEQ ID NO: 25.

14. The isolated antigen-binding protein of any of claims 1-13, which comprises H-FR1 comprising an amino acid sequence as set forth in SEQ ID NO: 46, H-FR2 comprising an amino acid sequence as set forth in SEQ ID NO: 47, H-FR3 comprising an amino acid sequence as set forth in SEQ ID NO: 48, and H-FR4 comprising an amino acid sequence as set forth in SEQ ID NO: 49.

15. The isolated antigen-binding protein of any one of claims 1-14, which comprises H-FR1 comprising an amino acid sequence as set forth in any one of SEQ ID NO: 5 and SEQ ID NO: 22, H-FR2 comprising an amino acid sequence as set forth in any one of SEQ ID NO: 6 and SEQ ID NO: 23, H-FR3 comprising an amino acid sequence as set forth in any one of SEQ ID NO: 7 and SEQ ID NO: 24, and H-FR4 comprising an amino acid sequence as set forth in any one of SEQ ID NO: 8 and SEQ ID NO: 25.

16. The isolated antigen-binding protein of claim 15, wherein the H-FR1, H-FR2, H-FR3, and H-FR4 comprise amino acid sequences selected from any one of the groups consisting of:
a) H-FR1: SEQ ID NO: 5, H-FR2: SEQ ID NO: 6, H-FR3: SEQ ID NO: 7, and H-FR4: SEQ ID NO: 8;
b) H-FR1: SEQ ID NO: 22, H-FR2: SEQ ID NO: 23, H-FR3: SEQ ID NO: 24, and H-FR4: SEQ ID NO: 25.

17. The isolated antigen-binding protein of any one of claims 1-16, comprising a heavy chain variable region VH comprising an amino acid sequence as set forth in SEQ ID NO: 50.

18. The isolated antigen-binding protein of claim 17, wherein the VH comprises an amino acid sequence as set forth in any one of SEQ ID NO: 1 and SEQ ID NO: 21.

19. The isolated antigen-binding protein of any one of claims 1-18, comprising LCDR3 comprising an amino acid sequence as set forth in SEQ ID NO: 12.

20. The isolated antigen-binding protein of any one of claims 1-19, comprising LCDR2 comprising an amino acid sequence as set forth in SEQ ID NO: 45.

21. The isolated antigen-binding protein of any one of claims 1-20, comprising LCDR2 comprising an amino acid sequence as set forth in any one of SEQ ID NO: 11 and SEQ ID NO: 28.

22. The isolated antigen-binding protein of any one of claims 1-21, comprising LCDR1 comprising an amino acid sequence as set forth in SEQ ID NO: 44.

23. The isolated antigen-binding protein of any one of claims 1-22, comprising LCDR1 comprising an amino acid sequence as set forth in any one of SEQ ID NO: 10 and SEQ ID NO: 27.

24. The isolated antigen-binding protein of any of claims 1-23, comprising a light chain variable region VL which comprises the LCDR1 comprising an amino acid sequence as set forth in SEQ ID NO: 44, the LCDR2 comprising an amino acid sequence as set forth in SEQ ID NO: 45, and the LCDR3 comprising an amino acid sequence as set forth in SEQ ID NO: 12.

25. The isolated antigen-binding protein of any of claims 1-24, comprising a light chain variable region VL which comprises the LCDR1 comprising an amino acid sequence as set forth in any one of SEQ ID NO: 10 and SEQ ID NO: 27, the LCDR2 comprising an amino acid sequence as set forth in any one of SEQ ID NO: 11 and SEQ ID NO: 28, and the LCDR3 comprising an amino acid sequence as set forth in SEQ ID NO: 12.

26. The isolated antigen-binding protein of claim 25, wherein the LCDR1, LCDR2, and LCDR3 comprise amino acid sequences selected from any one of the groups consisting of:
a) LCDR1: SEQ ID NO: 10, LCDR2: SEQ ID NO: 11, and LCDR3: SEQ ID NO: 12;
b) LCDR1: SEQ ID NO: 27, LCDR2: SEQ ID NO: 28, and LCDR3: SEQ ID NO: 12.

27. The isolated antigen-binding protein of any one of claims 1-26, comprising L-FR1, wherein the C-terminus of the L-FR1 is directly or indirectly linked to the N-terminus of the LCDR1, and the L-FR1 comprises an amino acid sequence as set forth in SEQ ID NO: 52.

28. The isolated antigen-binding protein of claim 27, wherein the L-FR1 comprises an amino acid sequence as set forth in any one of SEQ ID NO: 13 and SEQ ID NO: 29.

29. The isolated antigen-binding protein of any one of claims 1-28, comprising L-FR2, wherein the L-FR2 is located between the LCDR1 and the LCDR2, and the L-FR2 comprises an amino acid sequence as set forth in SEQ ID NO: 14.

30. The isolated antigen-binding protein of any one of claims 1-29, comprising L-FR3, wherein the L-FR3 is located between the LCDR2 and the LCDR3, and the L-FR3 comprises an amino acid sequence as set forth in SEQ ID NO: 53.

31. The isolated antigen-binding protein of claim 30, wherein the L-FR3 comprises an amino acid sequence as set forth in any one of SEQ ID NO: 15 and SEQ ID NO: 30.

32. The isolated antigen-binding protein of any one of claims 1-31, comprising L-FR4, wherein the N-terminus of the L-FR4 is directly or indirectly linked to the C-terminus of the LCDR3, and the L-FR4 comprises an amino acid sequence as set forth in SEQ ID NO: 54.

33. The isolated antigen-binding protein of claim 32, wherein the L-FR4 comprises an amino acid sequence as set forth in any one of SEQ ID NO: 16 and SEQ ID NO: 31.

34. The isolated antigen-binding protein of any of claims 1-33, comprising L-FR1 comprising an amino acid sequence as set forth in SEQ ID NO: 52, L-FR2 comprising an amino acid sequence as set forth in SEQ ID NO: 14, L-FR3 comprising an amino acid sequence as set forth in SEQ ID NO: 53, and L-FR4 comprising an amino acid sequence as set forth in SEQ ID NO: 54.

35. The isolated antigen-binding protein of any of claims 1-34, comprising L-FR1 comprising an amino acid sequence as set forth in any one of SEQ ID NO: 13 and SEQ ID NO: 29, L-FR2 comprising an amino acid sequence as set forth in SEQ ID NO: 14, L-FR3 comprising an amino acid sequence as set forth in any one of SEQ ID NO: 15 and SEQ ID NO: 30, and L-FR4 comprising an amino acid sequence as set forth in any one of SEQ ID NO: 16 and SEQ ID NO: 31.

36. The isolated antigen-binding protein of claim 35, wherein the L-FR1, L-FR2, L-FR3, and L-FR4 comprise amino acid sequences selected from any one of the groups consisting of:
a) L-FR1: SEQ ID NO: 13, L-FR2: SEQ ID NO: 14, L-FR3: SEQ ID NO: 15, and H-FR4: SEQ ID NO: 16;
b) L-FR1: SEQ ID NO: 29, L-FR2: SEQ ID NO: 14, L-FR3: SEQ ID NO: 30, and H-FR4: SEQ ID NO: 31.

37. The isolated antigen-binding protein of any one of claims 1-36, comprising a light chain variable region VL comprising an amino acid sequence as set forth in SEQ ID NO: 51.

38. The isolated antigen-binding protein of any one of claims 1-37, comprising a light chain variable region VL comprising an amino acid sequence as set forth in any one of SEQ ID NO: 9 and SEQ ID NO: 26.

39. The isolated antigen-binding protein of any one of claims 1-38, comprising a heavy chain constant region comprising an IgG-derived constant region or an IgY-derived constant region.

40. The isolated antigen-binding protein of claim 39, wherein the heavy chain constant region comprises a human IgG4-derived constant region.

41. The isolated antigen-binding protein of any one of claims 39-40, wherein the heavy chain constant region comprises an amino acid sequence as set forth in SEQ ID NO: 17.

42. The isolated antigen-binding protein of any one of claims 1-41, comprising a light chain constant region, and wherein the antibody light chain constant region comprises an Igκ-derived constant region or an Igλ-derived constant region.

43. The isolated antigen-binding protein of claim 42, wherein the light chain constant region comprises a human Igκ-derived constant region.

44. The isolated antigen-binding protein of any one of claims 42-43, wherein the light chain constant region comprises an amino acid sequence as set forth in SEQ ID NO: 18.

45. The isolated antigen-binding protein of any one of claims 1-44, comprising a heavy chain HC comprising an amino acid sequence as set forth in any one of SEQ ID NO: 19 and SEQ ID NO: 32.

46. The isolated antigen-binding protein of any one of claims 1-45, comprising a light chain LC comprising an amino acid sequence as set forth in any one of SEQ ID NO: 20 and SEQ ID NO: 33.

47. The isolated antigen-binding protein of any one of claims 1-46, which comprises HC comprising an amino acid sequence as set forth in any one of SEQ ID NO: 19 and SEQ ID NO: 32, and LC comprising an amino acid sequence as set forth in any one of SEQ ID NO: 20 and SEQ ID NO: 33.

48. The isolated antigen-binding protein of claim 47, wherein the HC and LC comprise amino acid sequences selected from any one of the groups consisting of:
a) HC: SEQ ID NO: 19 and LC: SEQ ID NO: 20;
b) HC: SEQ ID NO: 32 and LC: SEQ ID NO: 33.

49. The isolated antigen-binding protein of any one of claims 1-48, comprising an antibody or an antigen-binding fragment thereof.

50. The isolated antigen-binding protein of claim 49, wherein the antigen-binding fragment comprises Fab, Fab', F(ab)2, Fv fragments, F(ab')2, scFv, di-scFv, VHH, and/or dAb.

51. The isolated antigen-binding protein of any one of claims 49-50, wherein the antibody is selected from the group consisting of: monoclonal antibodies, single chain antibodies, murine antibodies, chimeric antibodies, humanized antibodies, and fully human antibodies.

52. A polypeptide comprising the isolated antigen-binding protein of any one of claims 1-51.

53. An immunoconjugate comprising the isolated antigen-binding protein of any one of claims 1-51 or the polypeptide of claim 52.

54. The immunoconjugate of claim 53, further comprising a pharmaceutically acceptable therapeutic agent.

55. The immunoconjugate of claim 54, wherein the therapeutic agent is selected from the group of a cytotoxic agent and a cytostatic agent.

56. An isolated nucleic acid molecule encoding the isolated antigen-binding protein of any one of claims 1-51 or the polypeptide of claim 52.

57. A vector comprising the isolated nucleic acid molecule of claim 56.

58. A cell comprising the isolated antigen-binding protein of any one of claims 1-51, the polypeptide of claim 52, the immunoconjugate of any one of claims 53-55, the isolated nucleic acid molecule of claim 56, and/or the vector of claim 57.

59. A method of preparing the isolated antigen-binding protein of any one of claims 1-51 or the polypeptide of claim 52, the method comprising culturing the cell of claim 58 under conditions capable of expressing the isolated antigen-binding protein of any one of claims 1-51 or the polypeptide of claim 52.

60. A pharmaceutical composition comprising the isolated antigen-binding protein of any one of claims 1-51, the polypeptide of claim 52, the immunoconjugate of any one of claims 53-55, the isolated nucleic acid molecule of claim 56, the vector of claim 57, the cell of claim 58, and/or a pharmaceutically acceptable adjuvant and/or excipient.

61. A pharmaceutical combination comprising the isolated antigen-binding protein of any one of claims 1-51 and an immune checkpoint inhibitor.

62. The pharmaceutical combination of claim 61, wherein the immune checkpoint inhibitor comprises a substance that inhibits the interaction of PD-1/PD-L1.

63. The pharmaceutical combination of any one of claims 61-62, wherein the immune checkpoint inhibitor is selected from the group consisting of: PD-1/PD-L1 blocking agents, PD-1 antagonists, PD-L1 antagonists, PD-1 inhibitors, and PD-L1 inhibitors.

64. The pharmaceutical combination of any one of claims 61-63, wherein the immune checkpoint inhibitor comprises an anti-PD-L1 antibody.

65. The pharmaceutical combination of claim 64, wherein the anti-PD-L1 antibody comprises HCDR3 comprising an amino acid sequence as set forth in SEQ ID NO: 37.

66. The pharmaceutical combination of any one of claims 64-65, wherein the anti-PD-L1 antibody comprises HCDR2 comprising an amino acid sequence as set forth in SEQ ID NO: 36.

67. The pharmaceutical combination of any one of claims 64-66, wherein the anti-PD-L1 antibody comprises HCDR1 comprising an amino acid sequence as set forth in SEQ ID NO: 35.

68. The pharmaceutical combination of any one of claims 64-67, wherein the anti-PD-L1 antibody comprises a heavy chain variable region VH which comprises HCDR1 comprising an amino acid sequence as set forth in SEQ ID NO: 35, HCDR2 comprising an amino acid sequence as set forth as SEQ ID NO: 36, and HCDR3 comprising an amino acid sequence as set forth in SEQ ID NO: 37.

69. The pharmaceutical combination of any one of claims 64-68, wherein the anti-PD-L1 antibody comprises a heavy chain variable region VH comprising an amino acid sequence as set forth in SEQ ID NO: 34.

70. The pharmaceutical combination of any one of claims 64-69, wherein the anti-PD-L1 antibody comprises LCDR3 comprising an amino acid sequence as set forth in SEQ ID NO: 41.

71. The pharmaceutical combination of any one of claims 64-70, wherein the anti-PD-L1 antibody comprises LCDR2 comprising an amino acid sequence as set forth in SEQ ID NO: 40.

72. The pharmaceutical combination of any one of claims 64-71, wherein the anti-PD-L1 antibody comprises LCDR1 comprising an amino acid sequence as set forth in SEQ ID NO: 39.

73. The pharmaceutical combination of any one of claims 64-72, wherein the anti-PD-L1 antibody comprises a light chain variable region VL which comprises LCDR1 comprising an amino acid sequence as set forth in SEQ ID NO: 39, LCDR2 comprising an amino acid sequence as set forth in SEQ ID NO: 40, and LCDR3 comprising an amino acid sequence as set forth in SEQ ID NO: 41.

74. The pharmaceutical combination of any one of claims 64-73, wherein the anti-PD-L1 antibody comprises a light chain variable region VL comprising an amino acid sequence as set forth in SEQ ID NO: 38.

75. The pharmaceutical combination of any one of claims 64-74, wherein the anti-PD-L1 antibody includes Atezolizumab.

76. Th pharmaceutical combination of any one of claims 61-75, which can be a pharmaceutical composition.

77. A kit comprising the pharmaceutical combination of any one of claims 61-76.

78. The isolated antigen-binding protein of any one of claims 1-51, the polypeptide of claim 52, the immunoconjugate of any one of claims 53-55, the isolated nucleic acid molecule of claim 56, the vector of claim 57, the cell of claim 58, and/or the pharmaceutical composition of claim 60 for the prevention, remission, and/or treatment of a tumor.

79. The use of claim 78, wherein the tumor includes a solid tumor.

80. The use of any one of claims 78-79, wherein the tumor includes a tumor associated with the expression of GARP protein.

81. The use of any one of claims 78-80, wherein the tumor includes a melanoma, a breast tumor, and/or a lung tumor.

82. The pharmaceutical combination of any one of claims 61-76 and/or the kit of claim 77 for the prevention, remission, and/or treatment of a tumor.

83. The use of claim 82, wherein the tumor includes a solid tumor.

84. The use of any one of claims 82-83, wherein the tumor includes a tumor associated with the expression of GARP.

85. The use of any one of claims 82-84, wherein the tumor includes a melanoma, a breast tumor, and/or a lung tumor.

86. Use of the isolated antigen-binding protein of any one of claims 1-51, the polypeptide of claim 52, the immunoconjugate of any one of claims 53-55, the isolated nucleic acid molecule of claim 56, the vector of claim 57, the cell of claim 58, and/or the pharmaceutical composition of claim 60 in the manufacture of a medicament for the prevention, remission, and/or treatment of a tumor.

87. The use of claim 86, wherein the tumor includes a solid tumor.

88. The use of any one of claims 86-87, wherein the tumor includes a tumor associated with the expression of GARP.

89. The use of any one of claims 86-88, wherein the tumor includes a melanoma, a breast tumor, and/or a lung tumor.

90. Use of a pharmaceutical combination of any one of claims 61-76 and/or a kit of claim 77 in the preparation of a medicament for the prevention, remission, and/or treatment of a tumor.

91. The use of claim 90, wherein the tumor includes a solid tumor.

92. The use of any one of claims 90-91, wherein the tumor includes a tumor associated with the expression of GARP.

93. The use of any one of claims 90-92, wherein the tumor includes a melanoma, a breast tumor, and/or a lung tumor.

94. A method for the prevention and/or treatment of a disease or disorder comprising administering to a subject in need thereof an effective amount of the isolated antigen-binding protein of any one of claims 1-51, the polypeptide of claim 52, the immunoconjugate of any one of claims 53-55, the isolated nucleic acid molecule of claim 56, the vector of claim 57, the cell of claim 58, the pharmaceutical composition of claim 60, wherein the disease or disorder includes a tumor.

95. The method of claim 94, wherein the tumor includes a solid tumor.

96. The use of any one of claims 94-95, wherein the tumor includes a tumor associated with the expression of GARP.

97. The method of any one of claims 94-96, wherein the tumor includes a melanoma, a breast tumor, and/or a lung tumor.

98. A method for the prevention and/or treatment of a disease or disorder comprising administering to a subject in need thereof an effective amount of the pharmaceutical combination of any one of claims 61-76, wherein the disease or disorder includes a tumor.

99. The method of claim 98, wherein the tumor includes a solid tumor.

100. The method of any one of claims 98-99, wherein the tumor includes a tumor associated with the expression of GARP.

101. The method of any one of claims 98-100, wherein the tumor includes a melanoma, a breast tumor, and/or a lung tumor.
